# FASCICULE DE BREVET EUROPEEN

(11) **EP 2 242 847 B1**
(45) Date de publication et mention de la délivrance du brevet: **25.11.2015**
(21) Numéro de dépôt: 09713778.0
(22) Date de dépôt: 22.01.2009
(51) Int. Cl.: C12N 15/85, A01K 67/027, C12N 5/10, C07K 16/00, C07K 16/46

(54) **SOURIS TRANSGENIQUES POUR LE GENE CGAMMA DES IMMUNOGLOBULINES HUMAINES DE CLASSE G**
TRANSGENMAUSE FÜR DAS CGAMMA-GEN DER MENSCHLICHEN G- IMMUNOGLOBULINE
TRANSGENIC MICE FOR THE CONSTANT REGION OF THE G-CLASS HUMAN IMMUNOGLOBULIN HEAVY CHAIN

(30) Priorité: 22.01.2008 FR 0800319
(43) Date de publication de la demande: 27.10.2010
(73) Titulaire: Université de Limoges, 87032 Limoges (FR); Centre National de la Recherche Scientifique, 75016 Paris (FR)
(72) Inventeur: COGNE, Michel, F-87170 Isle (FR); DUCHEZ, Sophie, 87350 Panazol (FR); COGNE, Nadine, F-87170 Isle (FR); PINAUD, Eric, F-87510 Saint-Jouvent (FR)
(74) Mandataire: Leblois-Préhaud, Hélène Marthe Georgette
(86) Numéro de dépôt international: PCT/FR2009/000068
(87) Numéro de publication internationale: WO 2009/106773

(56) Documents cités:
- WO-A-00/76310
- WO-A-93/12227
- WO-A-2005/047333
- US-A1- 2006 134 780
- GREEN L L: "Antibody engineering via genetic engineering of the mouse: XenoMouse strains are a vehicle for the facile generation of therapeutic human monoclonal antibodies" JOURNAL OF IMMUNOLOGICAL METHODS, ELSEVIER SCIENCE PUBLISHERS B.V.,AMSTERDAM, NL, vol. 231, no. 1-2, 10 décembre 1999 (1999-12-10), pages 11-23, XP004187631 ISSN: 0022-1759 cité dans la demande
- LONBERG N ET AL: "ANTIGEN-SPECIFIC HUMAN ANTIBODIES FROM MICE COMPRISING FOUR DISTINCT GENETIC MODIFICATIONS" NATURE, NATURE PUBLISHING GROUP, LONDON, UK, vol. 368, no. 6474, 28 avril 1994 (1994-04-28), pages 856-859, XP000941636 ISSN: 0028-0836 cité dans la demande
- CHAUVEAU C ET AL: "Insertion of the IgH locus 3' regulatory palindrome in expression vectors warrants sure and efficient expression in stable B cell transfectants" GENE, ELSEVIER, AMSTERDAM, NL, vol. 222, no. 2, 1 novembre 1998 (1998-11-01), pages 279-285, XP004150057 ISSN: 0378-1119 cité dans la demande
- GREEN L L ET AL: "ANTIGEN-SPECIFIC HUMAN MONOCLONAL ANTIBODIES FROM MICE ENGINEERED WITH HUMAN IG HEAVY AND LIGHT CHAIN YACS" NATURE GENETICS, NATURE PUBLISHING GROUP, NEW YORK, US, vol. 7, no. 1, 1 mai 1994 (1994-05-01), pages 13-21, XP000953045 ISSN: 1061-4036 cité dans la demande
- KHAMLICHI A A ET AL: "Immunoglobulin class-switch recombination in mice devoid of any Smu tandem repeat" BLOOD, W.B.SAUNDERS COMPANY, ORLANDO, FL, vol. 103, no. 10, 15 mai 2004 (2004-05-15), pages 3828-3836, XP002319762 ISSN: 0006-4971
- Sophie RAYNAL-DUCHEZ: "THESE pour soutenir le grade de docteur de l'université de Limoges : Modèles transgéniques pour l'étude de la fonction des récepteurs des cellules B et de leur glycosylation", 19 October 2007 (2007-10-19), UNIVERSITE DE LIMOGES, Limoges, XP055108079, * page 115 - page 129 * * page 111 - page 112 * & , Retrieved from the Internet: URL:http://epublications.unilim.fr/theses/ 2007/raynal-duchez-sophie/raynal-duchez-so phie.pdf

## Description

La présente invention est relative à une souris transgénique pour la région constante de la chaîne lourde des immunoglobulines humaines de classe G et à ses applications pour la production d'anticorps humanisés de classe IgG, en particulier d'IgG1 humanisées.

Les immunoglobulines ou anticorps sont des molécules bifonctionnelles capables de lier un grand nombre d'antigènes par leurs domaines variables (VH et VL) et d'interagir, par leurs domaines constants, avec des cellules du système immunitaire (monocytes, macrophages) et des systèmes effecteurs de l'organisme (système du complément, ADCC, opsonisation). La structure des immunoglobulines comprend deux chaînes légères identiques et deux chaînes lourdes identiques reliées par des ponts disulfures. Il existe cinq principales classes d'immunoglobulines ou anticorps (IgA, IgD, IgE, IgG, IgM) ainsi que des sous-classes ou isotypes (par exemple, IgG1 à IgG4 chez l'homme) déterminées par le type de leur chaîne lourde. La classe des IgG, et en particulier la sous-classe des IgG1 chez l'homme, correspond aux anticorps les plus actifs.

En effet, les immunoglobulines G (IgG) constituent l'essentiel de la réponse secondaire aux antigènes. La liaison de l'IgG à l'antigène a un effet direct de neutralisation (toxines, virus). La plupart des sous-classes d'IgG et notamment les IgG1 chez l'homme, sont capables d'activer la cascade enzymatique du complément qui aboutit à la lyse des cellules cibles. Les IgG sont les anticorps les plus efficaces pour sensibiliser les cibles des réactions de cytotoxicité-dépendante d'anticorps (ADCC pour *Antibody*-*Dependent Cell Cytotoxicity*). Les macrophages et les neutrophiles possèdent des récepteurs de Fcγ (RFcγ) qui participent à la capture des complexes immuns (phénomène d'opsonisation). En outre, les IgG franchissent la barrière placentaire (chez l'homme).

Les IgG comprennent deux chaînes lourdes identiques (chaîne γ), d'isotype γ1 à γ4 (sous-classe IgG1 à IgG4) chez l'homme, associées par l'intermédiaire de ponts disulfures, à deux chaînes légères identiques d'isotype kappa (κ) ou lambda (λ).

La chaîne lourde γ, qui est spécifique de cette classe d'immunoglobulines, existe sous forme membranaire et sous forme sécrétée. La forme sécrétée comprend quatre domaines d'environ 110 acides aminés : un domaine variable VH et trois domaines constants CH1, CH2 et CH3, ainsi qu'une région charnière (« *hinge* » ou H) entre CH1 et CH2. La forme membranaire comprend en outre un domaine hydrophobe (m1) permettant l'ancrage de la protéine dans la membrane, ainsi qu'un domaine intracytoplasmique (m2). La région de la chaîne lourde correspondant aux domaines CH1, CH2, H, CH3 (forme sécrétée) ou CH1, CH2, H, CH3, m1, m2 (forme membranaire), est dénommée région constante par opposition à la région correspondant au domaine variable VH, qui est dénommée région variable.

Les chaînes légères κ et λ, qui sont communes à l'ensemble des classes et sous-classes d'immunoglobulines, comprennent deux domaines : un domaine variable (VL) et un domaine constant (CL). Chez l'Homme, l'expression des chaînes κ et λ est équivalente, alors que chez la souris l'expression du locus λ est très ; faible de telle sorte que 95 % des chaînes légères sont de type κ. La région de la chaîne légère correspondant au domaine CL est dénommée région constante par opposition à la région correspondant au domaine variable VL, qui est dénommée région variable.

Les gènes des immunoglobulines sont organisés en locus, un locus pour les chaînes lourdes (locus IgH) et un locus pour chacune des chaînes légères (locus lambda et kappa).

Les locus des chaînes légères comprennent chacun des gènes V et J codant pour le domaine variable et des gènes C codant pour le domaine constant ; au cours de la différenciation des lymphocytes B, un gène V est réarrangé avec un gène J et un gène C, et la région V subit en outre des mutations somatiques qui permettent de produire des anticorps de forte affinité pour l'antigène.

Le locus des chaînes lourdes comprend des gènes V, D et J codant pour le domaine variable et des gènes C (Cµ, Cδ, Cγ, Cε et Cα) codant pour les domaines constants des isotypes des différentes classes d'immunoglobulines ; chaque gène C, sauf Cδ, est précédé d'une séquence de commutation ou « switch « (S). Les gènes Cγ (Cγ1 à Cγ4 chez l'homme) contiennent des introns séparant les exons codant pour les domaines constants CH1, H, CH2, CH3 et les exons de membrane ml et m2. Au cours de la différenciation des lymphocytes B, un gène V est réarrangé avec un gène D et un gène J, et la région V subit également des mutations somatiques qui permettent de produire des anticorps de forte affinité pour l'antigène. En outre, alors que la réponse primaire à l'antigène est constituée principalement d'IgM, la réponse secondaire est associée au mécanisme de commutation de classe au cours duquel la séquence de commutation Sµ, située en amont de Cµ recombine avec une autre séquence de commutation conduisant ainsi la production d'une autre classe d'immunoglobuline (IgG, IgE ou IgA).

La diversité des anticorps produits en réponse à la stimulation par un antigène provient de la combinaison de plusieurs mécanismes : la multiplicité des gènes V, la mutation somatique de ces gènes V, la recombinaison somatique des gènes V et la recombinaison somatique des séquences de commutation.

Du fait de leur capacité de liaison aux antigènes qui est extrêmement spécifique et de leurs propriétés effectrices (neutralisantes, cytotoxiques), les anticorps monoclonaux et en particulier ceux de la classe des IgG, possèdent un vaste champ d'applications dans de nombreux domaines, notamment médical, pour le diagnostic et le traitement de pathologies (pathologies infectieuses, tumorales, allergies, rejets de greffe, intoxications...).

Toutefois, l'utilisation *in vivo* chez l'homme d'anticorps monoclonaux à des fins diagnostiques (imagerie) ou thérapeutique est limitée du fait que la plupart des anticorps utilisables, sont des anticorps xénogéniques (anticorps de souris) qui sont mal tolérés et peu efficaces chez l'Homme et qu'il n'existe pas de méthode permettant d'obtenir des anticorps recombinants humains ou humanisés qui soient d'emblée majoritairement de la classe IgG, qui est la plus active.

En effet, l'utilisation d'anticorps de souris en immunothérapie humaine conduit souvent à l'apparition d'anticorps humains anti-anticorps de souris, phénomène pouvant s'accompagner de réactions d'hypersensibilité et de l'apparition de taux importants de complexes immuns en sus des capacités bloquantes de ces anticorps vis-à-vis de l'anticorps monoclonal. De plus les propriétés effectrices des anticorps: fixation au C1q, première étape de l'activation de la voie classique du complément qui aboutit à la lyse des cellules cibles, ou aux RFc, responsables de la capture des complexes immuns ou de la cytotoxicité-dépendante d'anticorps (ADCC), ne sont pas optimales pour les anticorps de souris injectés chez l'homme.

Les problèmes observés avec les anticorps monoclonaux de souris sont résolus par l'utilisation d'anticorps humanisés et en particulier d'IgG humanisées, de structure très proche des anticorps humains et donc mieux tolérés et plus efficaces. On appelle anticorps humanisé, un anticorps dérivé d'un mammifère non-humain par fusion des domaines constants des chaînes lourdes et légères d'un anticorps humain avec les domaines variables des chaînes lourdes et légères d'un anticorps d'un mammifère non-humain.

Toutefois, les méthodes de production d'anticorps recombinants humains ou humanisés de classe IgG actuellement disponibles sont peu efficaces et présentent les inconvénients suivants :
* l'immortalisation de lymphocytes B humains spécifiques d'un antigène est peu efficace,
* les méthodes *in vitro* reposent sur la production d'anticorps animaux, suivie du clonage des gènes codant leurs régions variables, de leur association par génie génétique avec des gènes correspondant aux régions constantes des anticorps humains, puis de l'expression *in vitro* dans des cellules CHO transfectées. Ces méthodes ne permettent pas de prédire l'activité potentielle des anticorps animaux chez l'homme du fait de leur structure qui est différente de celle des anticorps humains et de leur faible capacité à recruter des cellules effectrices humaines. A cause de ce problème, ce n'est qu'après un long travail d'immunisation *in vitro* qu'un tel anticorps peut être validé; les étapes de clonage et de construction des gènes chimériques sont longues et soumises à des aléas ; l'expression *in vitro* dans des cellules CHO transfectées a un rendement peu élevé.
* les méthodes *in vivo* reposent sur la production d'immunoglobulines monoclonales humaines directement chez des souris transgéniques dans lesquelles tout ou partie des loci des gènes d'immunoglobulines de souris sont remplacés par tout ou partie des loci équivalents humains : XenoMouse® d'Abgenix (Brevets US 6,114,598 ; 6,162,963 : 6,150,584 ; 6,673,986 ; 6,395,514 et EP 1 690 935 ; Demandes US 2005/287630 et 2005/241006), HuMAb-Mouse® de Medarex (Demandes US 2003/14483 et Brevet CA 2508763) ; Brevet US 5,545,807 ; TC Mouse™ de Kirin Brewery (Demande US 2002/0199213) et KM-Mouse® de Medarex et Kirin Brewery.

Initialement, deux types de souris transgéniques, permettant par croisement d'obtenir des souris productrices d'anticorps humains fonctionnels, ont été créées (Lonberg et al., Nature, 1994, 368 :856-859 ; Green et al., Nat. Genet., 1994, 7, 13-21). Les unes avaient intégré un fragment de 80 kb artificiellement reconstruit correspondant à diverses parties du locus des chaînes lourdes humaines (soit 4 gènes VH, 15 segments D, 6 segments J, les séquences codantes pour les régions Fcµ et Fcγ1 ainsi que celles nécessaires à la commutation); les autres avaient intégré un fragment de 43 kb contenant 4 gènes Vκ, 5 segments Jκ, le gène Cκ et les séquences enhancers adéquates. Ces lignées de souris transgéniques sont généralement déficientes pour la production d'immunoglobulines murines, en particulier du fait de l'invalidation du locus kappa endogène (souris κ -/-) et/ou d'une mutation qui inactive le locus IgH endogène (mutation µMT -/-). Les souris obtenues par croisement produisaient des anticorps humains de type IgM, ou IgG lors de réponses secondaires, indiquant que les fragments introduits permettent la commutation de classe; de plus, l'analyse des anticorps monoclonaux obtenus montra que des mutations somatiques affectaient les régions variables de ces anticorps lors de la réponse secondaire, indiquant l'existence d'une maturation apparemment correcte de la réponse anticorps.

D'autres lignées de souris comprenant les gènes Cµ, Cδ et l'un des gènes Cγ1, Cγ2, Cγ3 ou Cγ4 humains [IgG1, IgG2 et IgG4 : XenoMouse® d'Abgenix (Green et al., J. Immunol. Methods, 1999, 231, 11-23 ; Demande Internationale PCT WO 00/76310 et Demande américaine US 2006/0134780); IgG1 et IgG3 : HuMab Mouse® de GenPharm-Medarex] ou le locus de la chaîne légère lambda (Demande US 2002/0088016)) ont également été obtenues.

Une autre approche consistant à introduire des fragments chromosomiques humains entiers (technologie "transchromosomique"), a permis de générer des souris contenant 100 % des gènes humains (TC Mouse™ de Kirin Brewery ; Tomizuka et al., Proc. Natl. Acad. Sci., 2000, 97, 722-727). Des lignées de souris hybrides (KM-Mouse® : HuMab-Mouse® x TC Mouse™) ont été ensuite établies et présenteraient l'avantage de développer des réponses immunitaires beaucoup plus élevées que celles observées avec les souris HuMab-Mouse®.

Ces méthodes *in vivo* ne permettent pas de cibler la production des immunoglobulines humaines vers la classe IgG. Les modèles de souris transgéniques pour des contigs du locus des chaînes lourdes humaines incluant quelques gènes variables et les gènes Cµ et Cγ qui codent pour les régions constantes des anticorps humains IgM et IgG ont un répertoire de faible diversité du fait de l'inclusion partielle du locus au sein du contig, avec un nombre limité de gènes V, et donc des difficultés à obtenir des anticorps de haute affinité et à obtenir des IgG, chez des animaux qui produisent surtout des IgM humaines.

Par ailleurs, la Demande Internationale PCT WO 2005/04733 décrit une lignée de souris transgéniques produisant des quantités importantes d'immunoglobulines humanisées de classe IgA (de l'ordre du gramme par litre). Dans cette lignée, le gène Cα1 codant pour la chaîne lourde d'immunoglobuline α1 humaine a été inséré dans le locus IgH en lieu et place de la séquence de commutation Sµ (mutation alpha1 *knock*-*in* ou alpha1-KI). Dans cette construction, la suppression de la séquence de commutation Sµ associée à l'insertion du transgène Cα en lieu et place de cette séquence, abolit l'expression du gène µ endogène responsable de la synthèse de chaînes lourdes d'IgM. En outre, celle des autres gènes de chaînes lourdes d'immunoglobulines (IgG, IgE) est fortement diminuée du fait du blocage de la commutation de classe vers les gènes constants d'immunoglobulines situés en aval de Cµ sur le locus IgH endogène. Ainsi, les animaux transgéniques obtenus produisent des quantités importantes d'IgA chimériques dont le domaine constant de la chaîne lourde est humanisé et les domaines variables sont d'origine murine.

Toutefois, l'état actuel des connaissances ne permet pas d'envisager de produire d'autres classes d'immunoglobulines humaines par une stratégie analogue à celle décrite pour les IgA dans la Demande Internationale PCT WO 2005/047333. En effet, chaque classe d'immunoglobuline (IgA, IgG, IgE, IgM) constitue un récepteur différent, inséré dans la membrane cellulaire par un domaine de séquence différente. Par conséquent, l'efficacité des IgA humaines à permettre la différenciation B ne permet pas de déduire qu'il en serait de même pour n'importe qu'elle autre classe d'immunoglobuline humaine. De plus, la tentative d'expression d'IgE humaines en lieu et place des IgM de souris, suivant une stratégie analogue à celle utilisée pour les IgA, a résulté en l'absence complète de cellules B chez les animaux homozygotes pour la mutation. En outre, la différenciation des lymphocytes B par l'expression d'une immunoglobuline humaine de classe G chez la souris n'est pas connue.

Dans ce contexte, les Inventeurs ont construit une lignée de souris chez lesquelles le gène Cγ1 codant pour la chaîne lourde d'immunoglobuline γ1 humaine a été inséré dans le locus IgH en lieu et place de la séquence de commutation Sµ (mutation gamma1 *knock-in,* gamma1-KI ou γ1-KI). Ils ont montré que de façon imprévisible, l'obtention de mutants homozygotes pour l'insertion γ1 dans le locus IgH permettait d'obtenir des souris produisant des lymphocytes B qui expriment très majoritairement la chaîne lourde y1 humaine et à un taux très réduit (100 fois inférieur à la normale) les chaînes lourdes murines. Cette lignée dénommée "gammaprim", a été caractérisée comme capable de produire des IgG1 humanisées au niveau de la chaîne lourde et comme capable de produire des anticorps spécifiques après immunisation. Cette lignée est utilisée pour la production d'anticorps monoclonaux humanisés de classe IgG1 à des fins d'immunothérapie (anti-infectieuse ou anti-tumorale) ou de diagnostic. Cette lignée peut également être utilisée pour le criblage rapide d'anticorps monoclonaux ayant les propriétés effectrices d'une IgG1 humaine dans des tests d'activité *in vitro* ou *in vivo*.

En conséquence, l'invention a pour objet une souris transgénique, caractérisée en ce qu'elle comprend un locus IgH modifié par remplacement de la séquence de commutation Sµ par tout ou partie d'un transgène constitué par le gène Cγ d'une immunoglobuline humaine de classe G, incluant au moins l'exon codant pour le domaine CH3 et les exons de membrane m1 et m2 et en ce qu'elle produit des anticorps qui sont majoritairement des IgG chimériques dont les domaines constants des chaînes lourdes sont d'origine humaine.

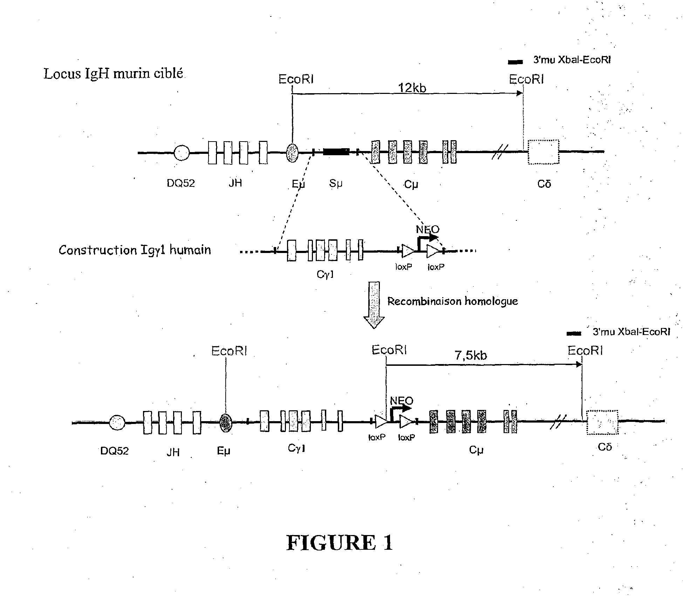

Conformément à l'invention, le transgène Cγ ou la partie de ce transgène incluant au moins l'exon codant pour le domaine CH3 et les exons de membrane m1 et m2, qui est inséré en lieu et place de la séquence de commutation Sµ, est donc situé entre l'activateur intronique Eµ, en 5' et le gène Cµ en 3'(figure 1).

Dans cette construction, la suppression de la séquence de commutation Sµ associée à l'insertion du transgène Cγ en lieu et place de cette séquence, abolit l'expression du gène µ endogène responsable de la synthèse de chaînes lourdes d'IgM. En outre, celle des autres gènes de chaînes lourdes d'immunoglobulines est également très fortement réduite du fait du blocage de la commutation de classe vers les gènes constants d'immunoglobulines situés en aval de Cµ sur le locus IgH endogène. Ainsi, les souris transgéniques obtenues produisent des quantités importantes d'IgG chimériques dont le domaine constant de la chaîne lourde est humanisé et les domaines variables sont d'origine murine.

La chaîne lourde transgénique γ humaine bénéficie d'un répertoire totalement diversifié puisque correspondant au répertoire normal généré par les réarrangements des segments VH, D et JH du locus IgH murin. En outre, les souris transgéniques sont capables de produire des anticorps humanisés de forte affinité en réponse secondaire à l'antigène, du fait que leurs lymphocytes B peuvent recruter le phénomène d'hypermutation somatique. Il en résulte l'obtention directe d'anticorps monoclonaux par immunisation des souris et dérivation d'hybridomes, sans nécessité de clonage des gènes d'immunoglobulines. L'activité effectrice (cytotoxique, neutralisante) des anticorps monoclonaux ainsi obtenus peut être validée immédiatement, et ces anticorps monoclonaux peuvent rapidement progresser vers le stade des essais cliniques ou d'une utilisation en tant que réactif de diagnostic. Les souris transgéniques selon l'invention constituent donc un outil précieux pour la production directe, le criblage et la validation fonctionnelle d'anticorps monoclonaux à visée thérapeutique ou diagnostique.

Selon un mode de réalisation avantageux de l'invention, ladite souris transgénique est homozygote pour ledit locus IgH modifié.

Selon un autre un mode de réalisation avantageux de l'invention, ledit locus IgH est modifié par remplacement de la séquence de commutation Sµ par la totalité du gène Cγ, incluant les exons CH1, charnière (H ou "*hinge*"), CH2, CH3 et les exons de membrane (m1 et m2), séparés par les introns correspondants.

Selon un autre un mode de réalisation avantageux de l'invention, ledit locus IgH est modifié par remplacement de la séquence de commutation Sµ, par le segment du gène Cγ incluant l'exon codant pour le domaine CH3 et les exons de membrane m1 et m2, séparés par les introns correspondants.

Selon un autre un mode de réalisation avantageux de l'invention, ledit gène Cγ est le gène Cγ1.

Selon encore un autre mode de réalisation avantageux de l'invention, ladite souris transgénique comprend un autre transgène codant pour une chaîne légère d'immunoglobuline humaine.

Selon une disposition avantageuse de ce mode de réalisation, le dit transgène est un gène kappa humain (codant pour une chaîne légère kappa humaine).

De préférence, ledit transgène est un gène kappa humain comprenant l'activateur intronique Eµ, en amont et le palindrome *hs3a*/*hs1,2*/*hs3b,* en aval. Ces séquences, qui sont décrites dans Chauveau et al., Gene, 1998, 222, 279-285, permettent d'obtenir une forte expression de la chaîne kappa humaine dans les cellules B et d'induire l'hypermutation somatique du transgène kappa humain: De manière préférée, ledit transgène est sous le contrôle du promoteur de la chaîne lourde humaine (pVH). Selon une disposition avantageuse de ce mode de réalisation, ledit gène kappa humain présente la séquence SEQ ID NO: 9.

Selon une autre disposition avantageuse de ce mode de réalisation, ledit mammifère non-humain transgénique est homozygote ou hétérozygote pour ledit transgène.

Selon une disposition avantageuse des modes de réalisation précédents de l'invention, lesdites souris transgéniques comprenant un autre transgène codant pour une chaîne légère kappa humaine possèdent un locus endogène de la chaîne légère kappa d'immunoglobulines inactivé (délété ou muté), notamment par recombinaison homologue. De préférence, lesdites souris transgéniques sont homozygotes pour ladite inactivation. Parmi les souris transgéniques dont le locus endogène de la chaîne légère kappa d'immunoglobuline a été inactivé par recombinaison homologue, on peut citer notamment la lignée de souris décrite dans Zou et al., EMBO J., 1993, 12, 811-820 et la mutation κD décrite dans Sirac et al., Proc. Natl., Acad. Sci. USA, 2006, 103, 7747-7752.

De telles souris transgéniques produisent des IgG humanisées dont la quasi-totalité des chaîne légères sont d'origine humaine.

L'invention englobe notamment une lignée de souris transgéniques, dénommée lignée "gammaprim", comprenant :
- un locus IgH modifié par remplacement de la séquence de commutation Sµ par le gène Cγ1 d'une immunoglobuline humaine de classe G.

L'invention englobe également une lignée de souris double-transgénique, dénommée lignée gammaprim/kappa, comprenant:
- un locus IgH modifié par remplacement de la séquence de commutation Sµ par le gène Cγ1 d'une immunoglobuline humaine de classe G, et
- un gène Vκ humain complet comprenant le gène Vκ1 réarrangé avec un gène Jκ5, l'intron Jκ-Cκ et le gène Cκ, sous le contrôle transcriptionnel du promoteur de la chaîne lourde humaine (pVH), de l'activateur intronique Eµ en amont, et du palindrome *hs3a*/*hs1,2*/*hs3b* en aval.

Les animaux de cette lignée double-transgénique produisent des IgG1 partiellement humanisées pour la chaîne lourde et totalement humanisées pour ce qui concerne la chaîne légère.

En effet, l'expression de la chaîne kappa transgénique dans cette lignée est capable d'entraîner l'exclusion allélique, c'est-à-dire d'empêcher dans la plupart des cellules B transgéniques, l'expression des gène endogènes de chaînes légères d'immunoglobulines murines.

Le répertoire de réponse aux antigènes de cette lignée de souris est normal étant donné que c'est essentiellement le domaine VH de la chaîne lourde qui contribue à la formation du site anticorps. Or, la chaîne lourde transgénique γ humaine bénéficie d'un répertoire totalement diversifié puisque correspondant au répertoire normal généré par les réarrangements des segments VH, D et JH du locus IgH murin, comme précisé ci-dessus.

En outre, les souris de cette lignée transgénique sont capables de produire des anticorps de forte affinité en réponse secondaire à l'antigène, du fait que leurs lymphocytes B peuvent recruter le phénomène d'hypermutation somatique, à la fois au niveau du gène de la chaîne lourde et du transgène de chaîne légère kappa.

Les souris transgéniques selon l'invention sont obtenues par les procédés classiques de transgénèse animale, selon les protocoles standards tels que décrits dans Transgenic Mouse: Methods and Protocols ; Methods in Molecular Biology, Clifton, N.J., Volume. 209, Octobre 2002. Edité par : Marten H. Hofker, Jan Van Deursen, Martern H. Hofker et Jan Van Deursen. Publié par Holly T. Sklar : Humana Press*.*

Les séquences des gènes humains et murins d'immunoglobulines qui servent à la construction des souris transgéniques selon l'invention sont connues et accessibles dans les bases de données; elles peuvent être amplifiées par PCR à l'aide d'amorces appropriées. Par exemple, la totalité du gène Cγ1 (SEQ ID NO: 13) incluant les séquences de l'exon CH1, de la région charnière (*"hinge"*)*,* des exons CH2, CH3 et des exons de membrane m1 et m2, séparées par les introns correspondants, peut être amplifiée par PCR à l'aide du couple d'amorces SEQ ID NO: 1 et 8.

La construction du gène Vκ humain est telle que décrite dans Chauveau et al., Gene, 1998, 222, 279-285 ; la séquence du gène Vκ1 réarrangé avec le gène Jκ5 et le gène Cκ correspond à la séquence GenBank X64133 (SEQ ID NO : 9) qui code pour une chaîne légère humaine présentant la séquence correspondant au numéro d'accès CAA45494 (SEQ ID NO : 10) dans la base de données EMBL.

Les insertions de fragments géniques dans le génome des souris peuvent être réalisées de façon aléatoire, de préférence elles sont réalisées de façon ciblée, par recombinaison homologue avec un vecteur de ciblage approprié comprenant éventuellement des séquences de recombinaison d'une recombinase site-spécifique comme les sites LoxP de la recombinase Cre. Les inactivations ou délétions de fragments géniques dans le génome des souris sont réalisées par recombinaison homologue avec un vecteur de ciblage approprié comprenant éventuellement des séquences de recombinaison d'une recombinase site-spécifique comme les sites LoxP de la recombinase Cre. Les souris double-transgéniques sont obtenus par croisement de souris transgéniques pour la chaîne lourde gamma avec des souris transgéniques pour la chaîne légère, tels que définis ci-dessus. La lignée gammaprim/kappa est obtenue par croisement de souris de la lignée gammaprim telle que définie ci-dessus avec des souris de la lignée κ ARN exprimant une chaîne légère kappa d'immunoglobuline humaine telle que décrite dans la Demande Internationale PCT WO 2005/047333.

Les souris double-transgéniques sont éventuellement croisées avec des souris transgéniques dont le locus endogène de la chaîne légère kappa d'immunoglobulines a été inactivé par recombinaison homologue, telles que définies ci-dessus.

La présente invention a également pour objet un vecteur de ciblage de la recombinaison homologue, caractérisé en ce qu'il comprend le gène Cγ d'une immunoglobuline humaine de classe G ou un segment de ce gène incluant au moins l'exon codant pour le domaine CH3 et les exons de membrane m1 et m2, flanqué de fragments de séquences du locus IgH murin qui sont adjacents à la séquence Sµ.

Selon un mode de réalisation avantageux dudit vecteur de ciblage. Il peut comprendre une cassette d'expression d'un marqueur de sélection approprié, adjacente audit gène Cγ ou au segment dudit gène tel que défini ci-dessus.

Ladite cassette d'expression peut être flanquée de séquences de recombinaison site-spécifique. De préférence, lesdites séquences sont les séquences LoxP de la recombinase Cre. Cette disposition permet éventuellement d'exciser ladite cassette d'expression.

Les fragments de séquences qui sont adjacents à la séquence Sµ sont d'origine murine.

Selon un autre mode de réalisation dudit vecteur de ciblage, le gène Cγ ou le segment dudit gène est flanqué en 5' d'un fragment d'environ 5 κb correspondant à la région JH/Eµ et en 3' d'un fragment d'environ 5 kb correspondant à la région Cµ, lesquels fragments correspondant respectivement aux positions 131281 à 136441 et 140101 à 145032 de la séquence GenBank AC073553 (SEQ ID NO : 11 et SEQ ID NO : 12).

La présente invention a également pour objet des cellules embryonnaires de souris, modifiées par un vecteur de ciblage tel que défini ci-dessus.

Les dites cellules embryonnaires (cellules souches totipotentes) modifiées sont utiles pour l'obtention des souris transgéniques tels que définies ci-dessus ; elles sont injectées dans des blastocystes de souris, selon les techniques classiques de transgénèse animale.

La présente divulgation a également pour objet, l'utilisation d'un mammifère non-humain transgénique tel que défini ci-dessus pour la production d'anticorps humanisés de classe IgG ou de fragments de ces anticorps.

La présente divulgation a également pour objet un procédé de préparation d'anticorps humanisés de classe IgG ou de fragments de ces anticorps, caractérisé en ce qu'il comprend au moins les étapes suivantes :
- l'immunisation d'une souris transgénique tel que défini ci-dessus avec un antigène d'intérêt,
- la préparation, par tout moyen approprié, d'anticorps humanisés de classe IgG ou de fragments de ces anticorps, à partir du sérum, des sécrétions ou des lymphocytes B de ladite souris transgénique.

La présente divulgation a également pour objet un procédé de criblage d'anticorps thérapeutiques, caractérisé en ce qu'il comprend au moins les étapes suivantes :
- l'immunisation d'une souris transgénique tel que défini ci-dessus avec un antigène d'intérêt,
- la préparation, par tout moyen approprié, d'anticorps monoclonaux dirigés contre ledit antigène d'intérêt, à partir des lymphocytes B de ladite souris transgénique,
- l'évaluation de l'activité desdits anticorps monoclonaux, par tout moyen approprié, et
- la sélection des anticorps monoclonaux actifs.

L'activité desdits anticorps monoclonaux est notamment l'activité de liaison spécifique de l'antigène, l'activité neutralisante vis-à-vis d'un pathogène (virus, champignon, bactérie, parasite) ou d'une toxine ou bien l'activité cytotoxique vis-à-vis de cellules tumorales ou de cellules infectées par un pathogène tel que définit ci-dessus. Cette activité est mesurée *in vitro* ou *in vivo* par des tests classiques bien connus de l'Homme du métier.

Les souris transgéniques selon l'invention présentent l'avantage de permettre la production d'anticorps monoclonaux de classe IgG qui sont d'emblée des anticorps chimériques humanisés de classe IgG. Le procédé de production d'anticorps monoclonaux humanisés de classe IgG selon l'invention est donc plus simple, plus rapide et plus économique que les procédés de l'art antérieur puisqu'il ne nécessite pas d'étapes additionnelles de clonage des gènes desdits anticorps et de fusion des domaines variables desdits anticorps avec les domaines constants d'immunoglobulines humaines. L'activité effectrice (cytotoxique, neutralisante) des anticorps monoclonaux ainsi obtenus peut être validée immédiatement par des tests d'activité *in vitro* ou *in vivo*. Ces anticorps monoclonaux peuvent rapidement progresser vers le stade des essais cliniques ou d'une utilisation en tant que réactif de diagnostic.

La souris englobe la production d'anticorps polyclonaux ou monoclonaux constitués par des IgG ou leurs fragments, notamment les fragments Fab, Fab'2 et Fc. De préférence, les dits fragments sont des fragments fonctionnels comprenant les domaines variables des chaînes lourdes et légères (Fab, Fab'2, Fv, scFv).

Les anticorps humanisés de classe IgG tels que définis ci-dessus et leurs fragments sont préparés par les techniques classiques connues de l'Homme du métier, telles que celles décrites dans Antibodies : A Laboratory Manual, E. Howell and D Lane, Cold Spring Harbor Laboratory, 1988*.*

De manière plus précisé : ,
- les anticorps polyclonaux sont préparés par immunisation d'une souris transgénique tel que définie ci-dessus avec un antigène d'intérêt, éventuellement couplé à la KLH ou à l'albumine et/ou associé à un adjuvant approprié tel que l'adjuvant de Freund (complet ou incomplet) ou l'hydroxyde d'alumine ; après obtention d'un titre en anticorps satisfaisant, les anticorps sont récoltés par prélèvement du sérum des souris immunisées et enrichis en IgG par précipitation, selon les techniques classiques, puis les IgG spécifiques sont éventuellement purifiées par chromatographie d'affinité sur une colonne appropriée sur laquelle est fixé l'antigène tel que défini ci-dessus, de façon à obtenir une préparation d'IgG monospécifiques.
- les anticorps monoclonaux sont produits à partir d'hybridomes obtenus par fusion de lymphocytes B d'un mammifère non-humain transgénique tel que défini ci-dessus avec des myélomes, selon la technique de Köhler et Milstein (Nature, 1975, 256, 495-497) ; les hybridomes sont cultivés *in vitro*, notamment dans des fermenteurs ou produits *in vivo,* sous forme d'ascite ; alternativement, lesdits anticorps monoclonaux sont produits par génie génétique comme décrit dans le brevet américain US 4,816,567. Par exemple, des souris transgéniques tels que définies ci-dessus sont immunisées de façon forte et répétée avec des antigènes choisis (antigènes de bactéries, de virus, de champignons, antigènes spécifiques de tumeurs tels que l'antigène carcino-embryonnaire, etc...), selon un protocole standard comprenant une première immunisation par injection intrapéritonéale de l'antigène dans un volume équivalent d'adjuvant complet de Freund puis une deuxième immunisation (rappel) 15 jours plus tard dans des conditions identiques mais avec cette fois de l'adjuvant incomplet de Freund. Les anticorps monoclonaux sont produits selon un protocole standard comprenant le sacrifice des souris deux semaines après le dernier rappel, le prélèvement de la rate, la mise en suspension des lymphocytes spléniques et la fusion de ces lymphocytes avec la lignée cellulaire SP2/0 (cette lignée murine ne produit aucun anticorps murin, est immortalisée, et possède toute la machinerie de sécrétion nécessaire à la sécrétion d'immunoglobulines).
- les fragments d'anticorps sont produits à partir des régions V_{H} et V_{L} clonées, à partir des ARNm d'hybridomes ou de lymphocytes spléniques d'une souris transgénique selon l'invention, immunisé ; par exemple, les fragments Fv ou Fab sont exprimés à la surface de phages filamenteux selon la technique de Winter et Milstein (Nature, 1991, 349, 293-299) ; après plusieurs étapes de sélection, les fragments d'anticorps spécifiques de l'antigène sont isolés et exprimés dans un système d'expression approprié, par les techniques classiques de clonage et d'expression d'ADN recombinant.

Les anticorps ou leur fragments tels que définis ci-dessus, sont purifiés par les techniques classiques connues de l'Homme du métier, telles que la chromatographie d'affinité.

La présente invention a également pour objet, un anticorps humanisé de classe IgG susceptible d'être obtenu par le procédé tel que défini ci-dessus à partir des lymphocytes B d'une souris transgénique, caractérisé en ce qu'il comprend des chaînes lourdes chimériques dont les domaines constants sont d'origine humaine et les domaines variables sont issus de ladite souris , et des chaînes légères humaines dont les domaines variables sont codés par Vκ1-Jκ5.

L'invention englobe les anticorps humanisés de classe IgG dont la chaîne légère est codée par le gène Vκ1-Jκ5 présentant la séquence Genbank X64133 (SEQ ID NO : 9) ou une séquence produite par hypermutation de cette séquence, notamment après activation des lymphocytes B en présence de l'antigène.

L'invention englobe les anticorps polyclonaux, les anticorps monoclonaux ainsi que leurs fragments (Fab, Fc, Fab'2) susceptible d'être obtenu par le procédé tel que défini ci-dessus, caractérisé en ce qu'il comprend un fragment desdites chaînes lourdes et légères telles que définies ci-dessus.

Les anticorps humanisés selon l'invention et leurs fragments tels que définis ci-dessus, sont bien tolérés chez l'Homme (minimisation du risque de réaction allergique par immunisation inter-espèce) et possèdent une demi-vie prolongée chez l'Homme, étant donné que la région constante de la chaîne lourde et la totalité de la chaîne légère de ces anticorps sont d'origine humaine.

La présente divulgation a également pour objet un anticorps humanisé de classe IgG ou un fragment de cet anticorps, tels que définis ci-dessus comme médicament ; un tel anticorps ou son fragment possédant des propriétés effectrices (neutralisante, cytotoxique) dirigées contre un antigène d'intérêt sont utilisés en immunothérapie pour la prévention et le traitement de pathologies telles que notamment les maladies infectieuses, les cancers, les intoxications, les allergies, les rejets de greffe.

La présente invention a également pour objet une composition pharmaceutique, caractérisée en ce qu'elle comprend au moins un anticorps humanisé de classe IgG ou un fragment de cet anticorps, tels que définis ci-dessus, associé par tout moyen approprié à une molécule thérapeutique; l'anticorps est de préférence dirigé contre un antigène membranaire, de façon à cibler le principe actif (toxine, agent antiviral, anticancéreux) dans la cellule cible.

Les compositions peuvent contenir en outre, au moins un véhicule pharmaceutiquement acceptable et éventuellement des substances porteuses et/ou des adjuvants.

Les véhicules pharmaceutiquement acceptables, les substances porteuses et les adjuvants sont ceux classiquement utilisés.

Les adjuvants sont avantageusement choisis dans le groupe constitué par des émulsions huileuses, de la saponine, des substances minérales, des extraits bactériens, de l'hydroxyde d'alumine et le squalène.

Les substances porteuses sont avantageusement sélectionnées dans le groupe constitué par des liposomes unilamellaires, des liposomes multilamellaires, des micelles de saponine ou des microsphères solides de nature saccharidique ou aurifère.

Les compositions selon l'invention, sont administrées par voie intramusculaire, sous-cutanée, intra-péritonéale, intraveineuse, intracranienne, orale, sublinguale, intranasale, oculaire, nasale, rectale, par inhalation ou par application transdermique; la dose et le rythme d'administration varient en fonction de l'espèce (humaine ou animale) et de la maladie à traiter.

La présente invention a également pour objet un réactif de diagnostic comprenant un anticorps humanisé de classe IgG ou un fragment de cet anticorps, tels que définis ci-dessus.

Les réactifs peuvent être couplés à des particules ou à un marqueur approprié. Les particules sont notamment des particules d'or, des liposomes (liposomes contenant un colorant) ou des érythrocytes (immunoagglutination). Le marqueur est une substance qui produit un signal détectable par les méthodes conventionnelles ou qui réagit avec une autre substance, de façon à produire un signal détectable. Parmi les marqueurs utilisables dans les méthodes de diagnostic selon l'invention, on peut citer notamment, les chromophores, les substances fluorescentes ou chimioluminescentes, les enzymes, les isotopes radioactifs et les agents complexants (Protéine A, Protéine G, biotine, lectine).

Les réactifs tels que définis ci-dessus peuvent également être immobilisés sur un support approprié, selon les méthodes classiques connues de l'homme du métier. Parmi les supports appropriés sur lesquels peuvent être immobilisés ces réactifs on peut citer notamment ceux en matière plastique (latex, polystyrène, poly-vinylchloride, polyuréthane, polyacrylamide, polyvinylacétate), en verre, ou en papier (nitrocellulose). Ces supports sont sous la forme de plaques (microplaques), de feuilles, de bandelette, ou de particules (billes). L'immobilisation des réactifs sur le support peut être réalisée par formation d'un pontage entre le réactif et une molécule qui est couplée au support (hydrazide, Protéine A, glutaraldéhyde, carboiimide ou lysine), selon les techniques conventionnelles.

Les réactifs selon l'invention sont utilisables dans des méthodes de diagnostic qui mettent en oeuvre des techniques d'immunochimie conventionnelles, par exemple : EIA, ELISA, RIA, immunofluorescence, immunoagglutination, immunochromatographie, immunocytochimie, immunohistochimie, immunoblot, et immunoprécipitation.

La présente divulgation a également pour objet l'utilisation d'un anticorps humanisé de classe IgG ou un fragment de cet anticorps, tels que définis ci-dessus, pour la préparation d'un médicament destiné à la prévention et au traitement d'une pathologie sélectionnée dans le groupe constitué par: les maladies infectieuses, les cancers, les intoxications, les allergies et les rejets de greffe. La présente divulgation a également pour objet l'utilisation d'un anticorps humanisé de classe IgG ou un fragment de cet anticorps, tels que définis ci-dessus, pour la préparation d'un réactif destiné au diagnostic d'une pathologie sélectionnée dans le groupe constitué par: les maladies infectieuses et les cancers.

Outre les dispositions qui précèdent, la divulgation comprend encore d'autres dispositions qui ressortiront de la description qui va suivre, qui se réfère à des exemples d'obtention et d'utilisation des souris transgéniques selon la présente invention ainsi qu'aux dessins annexés dans lesquels :
- la figure 1 illustre la structure du locus IgH modifié obtenu par recombinaison homologue entre le locus IgH murin et le vecteur de ciblage dénommé p-gamma1KI, comprenant : i) un fragment de 6860 pb du gène d'immunoglobuline gamma1 humain incluant les exons codant pour les domaines constants CH1, charnière ("*hinge*"), CH2 et CH3 et les exons de membrane m1 et m2 de la chaîne lourde d'immunoglobuline γ1, ii) une cassette néo bordée de sites LoxP, iii) des séquences flanquantes homologues à une portion du locus murin des chaînes lourdes d'immunoglobulines, avec, en amont, un fragment d'environ 5 kb correspondant à la région JH-Eµ (fragment DQ 52/JH) et en aval un autre fragment d'environ 5 kb correspondant au gène Cµ (fragment Cµ). Les sites *EcoR*I sont indiqués, ainsi que la position de la sonde (*3'muXba*I*-EcoR*I) s'hybridant en dehors du site de recombinaison homologue, en 5' du gène constant IgH Delta (δ) et de son site *EcoR*I. L'allèle recombinant est caractérisé par un fragment d'environ 7,5 kb représentant le fragment µ murin et la cassette néo alors que l'allèle sauvage est caractérisé par un fragment de 12 kb.

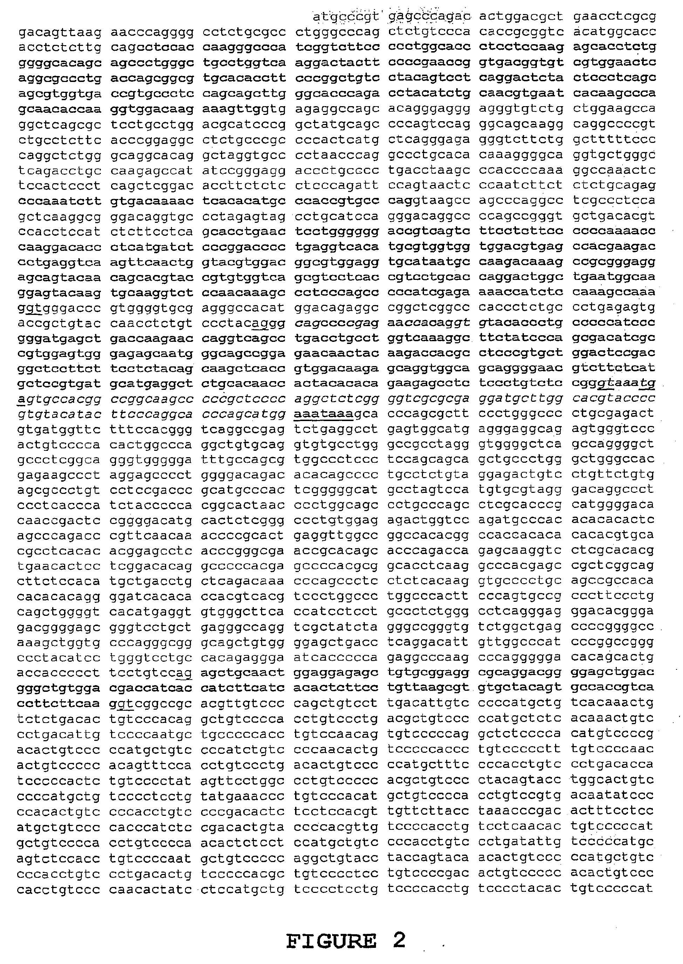

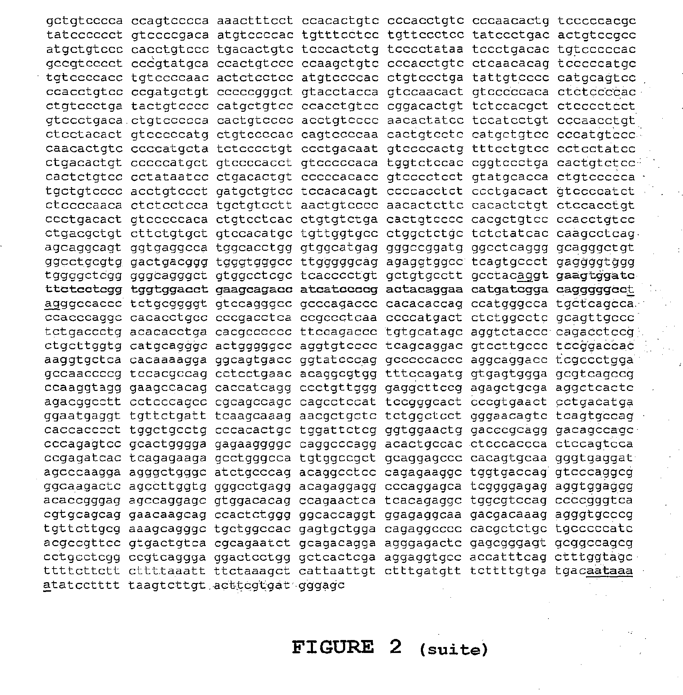

- la figure 2 représente la séquence du fragment de 6860 pb (SEQ ID NO : 13) du gène d'immunoglobuline gamma1 humain inséré au locus IgH des souris transgéniques gammaprim. Les exons codant pour les domaines constants CH1, charnière ("*hinge*"), CH2 et CH3 et les exons de membrane m1 et m2 de la chaîne lourde d'immunoglobuline γ1 sont indiqués en caractères gras. Les sites d'épissages 5' et 3' sont soulignés et les sites de polyadénylation sont indiqués en caractères gras et soulignés. Les séquences correspondant aux amorces Gam1CH1-5'F et Gam1 mbexon-3'R sont indiquées en grisé.

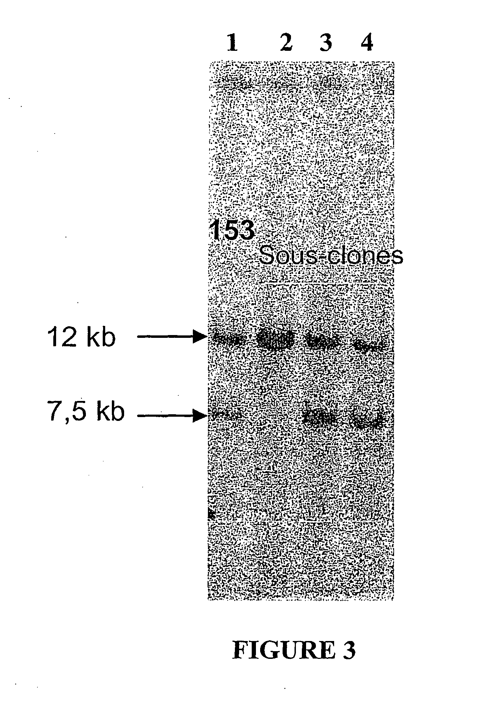

- la figure 3 illustre l'analyse en Southern-blot de l'ADN génomique de clones ES transfectés avec le vecteur de ciblage p-gamma1KI ; l'ADN génomique digéré par *EcoR*I est hybridé avec une sonde correspondant à la région 5' du gène δ. **1**. clone recombinant (n°153). **2.** ADN génomique sauvage. **3.** sous-clone issu du clone n°153. **4.** sous-clone issu du clone n°153. La présence d'un fragment de 7,5 kb indique que le clone a intégré le transgène γ1 humain par recombinaison homologue. Par comparaison, un fragment de 12 kb est présent dans l'ADN génomique sauvage.

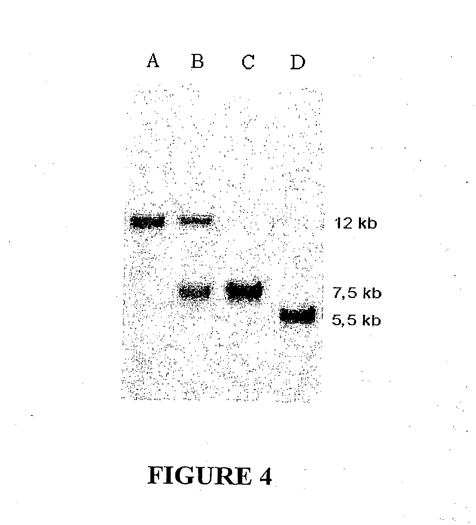

- la figure 4 illustre l'analyse en Southern-blot de l'ADN génomique de queue de souris; l'ADN génomique digéré par *EcoR*I est hybridé avec une sonde située en 5' du gène δ. **A**. souris sauvage (WT). **B**. hétérozygote γ1-KI. **C.** homozygote γ1-KI. **D.** homozygote γ1-KI "floxé" (excision du gène néo par la recombinase Cre). L'allèle recombinant correspond à un fragment d'environ 7,5 kb représentant le fragment µ murin et la cassette néo. L'allèle recombinant "floxé" correspond à un fragment d'environ 5,5 kb représentant le fragment µ murin seul (sans la cassette néo). L'allèle sauvage correspond à un fragment de 12 kb.

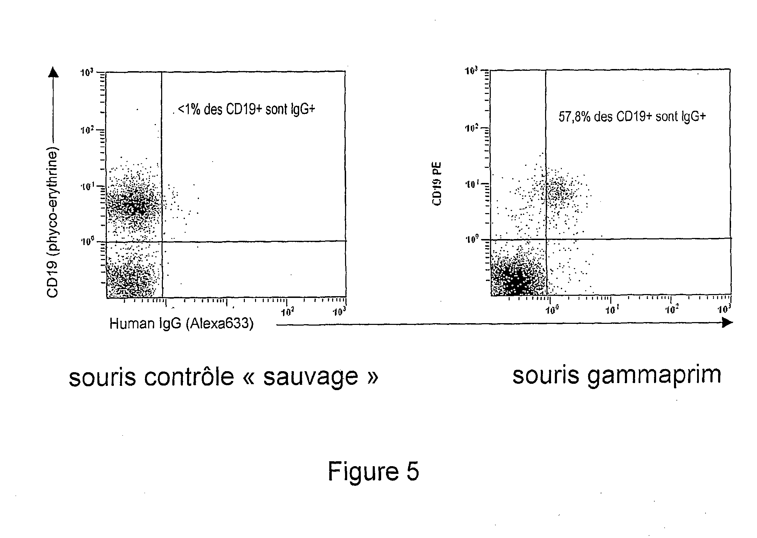

- la figure 5 illustre l'analyse en cytométrie de flux de l'expression mémbranaire des IgG1 humaines à la surface des lymphocytes spléniques d'un animal homozygote de la lignée transgénique gammaprim comparé à un animal contrôle. Les lymphocytes spléniques ont été marqués avec des anticorps spécifiques de CD19 (marqueur spécifique des cellules B) et d'IgG1 humaines. Pour l'analyse en cytométrie, une fenêtre a été établie sur les lymphocytes CD19+.

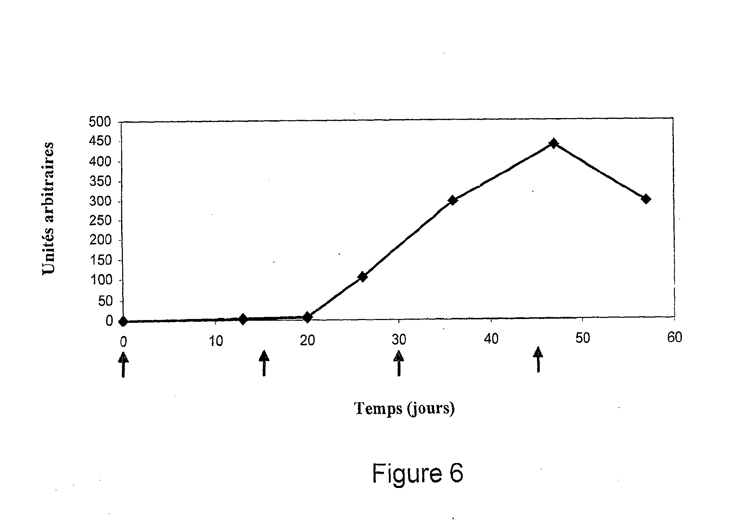

- la figure 6 illustre l'analyse en ELISA de la réponse en anticorps IgG1 humaines spécifiques, d'un animal de la lignée gammaprim immunisé par l'antigène p24 du VIH. Les résultats sont exprimés en unités arbitraires (UA) d'IgG1 anti-p24 en fonction du temps en jours (les flèches représentent la première immunisation à J0, puis les rappels).

### Exemple 1 : Obtention et caractérisation génotypique de la lignée de souris transgéniques homozygotes pour la mutation gammaprim

Le gène de la chaîne lourde d'immunoglobuline gamma1 humaine, incluant les exons codant pour les domaines CH1, charnière ("*hinge*"), CH2 et CH3 et les exons de membrane (m1 et m2), a été inséré par recombinaison homologue, en lieu et place de la région de commutation Sµ de la chaîne lourde murine (Sµ), de façon à bloquer la commutation de classe vers les gènes constants d'immunoglobulines situés en aval de Cµ sur le locus endogène (locus IgH murin, figure 1). La région ciblée abolit l'expression du gène µ endogène responsable de la synthèse de chaînes lourdes d'IgM, et diminue également de façon drastique celle des autres gènes de chaînes lourdes d'immunoglobulines. En conséquence, la lignée transgénique obtenue produit une forte quantité d'IgG chimériques dont le domaine constant humanisé correspond à l'isotype IgG1.

### 1) Construction du vecteur de ciblage de la recombinaison homologue

Les constructions plasmidiques ont été réalisées à partir du plasmide bluescript SK (pSK) (STRATAGENE) et de la souche bactérienne *E. coli* TG1 (STRATAGENE), en utilisant les protocoles classiques de préparation, de clonage et d'analyse de l'ADN tels que ceux décrits dans Current Protocols in Molecular Biology (Frederick M. AUSUBEL, 2000, Wiley and son Inc, Library of Congress, USA).

Le vecteur de recombinaison homologue ou vecteur de ciblage dérivé de pSK, dénommé p-gamma1KI (figure 1), comprend : i) un fragment de 6860 pb (SEQ ID NO : 13) du gène d'immunoglobuline gamma1 humain incluant les exons codant pour les domaines constants CH1, charnière ("*hinge*"), CH2 et CH3 et les exons de membrane m1 et m2 de la chaîne lourde d'immunoglobuline γ1 (figure 2), ii) une cassette néo (fragment de 1,6kb), iii) des séquences flanquantes homologues à une portion du locus murin des chaînes lourdes d'immunoglobulines, avec, en amont, un fragment d'environ 5 kb correspondant à la région JH-Eµ (fragment DQ 52/JH) et en aval un autre fragment d'environ 5 kb correspondant au gène Cµ (fragment Cµ).

De manière plus précise, les différents fragments ont été insérés dans le plasmide bluescript SK, selon les étapes suivantes :
- Dans une première étape, le fragment Cµ correspondant aux positions 140101 à 145032 de la séquence Genbank AC073553 (SEQ ID NO : 12) a été amplifié par PCR à l'aide d'amorces spécifiques appropriées puis cloné au site *Xho*I de pSK pour donner le plasmide pA.
- Dans une seconde étape, le fragment DQ 52/JH correspondant aux positions 131281 à 136441 de la séquence GenBank AC073553 (SEQ ID NO : 11) a été amplifié par PCR à l'aide d'amorces spécifiques appropriées puis clone en 5' du fragment Cµ, entre les sites *EcoR*V et *Cla*I du plasmide pA, pour donner le plasmide pB.
- Dans une troisième étape, la cassette néo décrite dans Pinaud et al., Immunity, 2001, 15, 187-199, a été insérée au site *Sal*I entre DQ52/JH et Cµ, pour donner le plasmide pC.
- Dans une quatrième étape, le fragment Cγ1 de 6860 pb flanqué d'adaptateurs *Cla*I et contenant le gène gamma1 humain a été amplifié en une seule étape de PCR grand fragment à l'aide du couple d'amorces **Gam1CH1-5'F** (5' caatcgatgcccgtgagcccagac 3', SEQ ID NO: 1) et **Gam1 mbexon-3'R** (5' aaatcgatgctcccatcacgaagtacaa 3', SEQ ID NO: 8) puis les séquences de l'ensemble des régions codantes et des sites d'épissage ont été vérifiées par PCR à l'aide des amorces suivantes:
   - **Gam1CH1-5'F** (SEQ ID NO: 1)
   - **Gam1 CH1-3'R ;** 5' gcttccagcagagacaccct 3' (SEQ ID NO: 2)
   - **Gam1 CH2-5'F :** 5' cagctcggacaccttctc 3' (SEQ ID NO: 3)
   - **Gam1 CH2-3'R :** 5'ccggcctctgtccatgtg 3' (SEQ ID NO: 4)
   - **Gam1 CH3-5'F :** 5'cacatggacagaggccgg 3' (SEQ ID NO: 5)
   - **Gam1 CH3-3'R :** 5'ctgacccgtggaaagaac 3' (SEQ ID NO: 6)
   - **Gam1 mbexon-5'F :** 5' agctgacctcaggacatt 3' (SEQ ID NO: 7)
   - **Gam1 mbexon-3'R** (SEQ ID NO: 8)

Enfin, dans une dernière étape, le fragment Cγ1 de 6860 pb flanqué d'adaptateurs *Cla*I et contenant le gène gamma1 humain ainsi obtenu, a été inséré entre le fragment JH et la cassette néo au site *Cla*I du plasmide pC pour donner le vecteur de ciblage dénommé p-gamma1KI.

La séquence de p-gamma1KI a été vérifiée par séquençage automatique et par analyse de restriction avec les enzymes *Cla*I et *Xho*I, confirmant que le vecteur était apte à permettre une recombinaison homologue telle que schématisée à la figure 1.

### 2) Transfection de cellules ES et injection dans des blastocystes

Des cellules ES ont été transfectées par électroporation de l'ADN de p-gamma1KI linéarisé au site *Not*I. Les clones sélectionnés en présence de généticine ont été prélevés et l'ADN génomique digéré par *EcoR*I a été analysé par Southern Blot à l'aide d'une sonde radioactive s'hybridant en dehors du site de recombinaison homologue, en 5' du gène constant IgH Delta (δ) et de son site *EcoR*I (figure 1) ; cette sonde dénommée 3'mu*Xba*I-*EcoR*I, amplifiée par PCR à l'aide d'amorces spécifiques appropriées, correspond aux positions 145032 à 145945 de la séquence Genbank AC073553.

La présence d'un allèle recombinant est visualisée par un fragment d'environ 7,5 kb (représentant le fragment µ murin et la cassette néo) alors que l'allèle sauvage correspond à un fragment de 12 kb (figure 3). Dans ces conditions, sur 192 clones analysés, 1 s'est révélé positif.

La vérification du caryotype de deux des quatre clones recombinants n'a montré aucune anomalie chromosomique (aneuploïdie).

Ce clone a été injecté dans des blastocystes de souris C57/Black 6 en utilisant les protocoles classiques de transgénèse tels que ceux décrits dans *Transgenic Mouse: Methods and Protocols,* précité. Un mâle chimère, présentant un degré de chimérisme élevé, a été obtenu et mis en accouplement avec des femelles C57/Black 6 afin d'obtenir des animaux hétérozygotes qui ont été analysés par Southern-Blot (Figure 4), PCR et ELISA. Une lignée de souris homozygotes pour le locus IgH recombiné (Figure 4), dénommée ci-après lignée gamma1 *knock*-*in*, gammal-KI, γ1-KI ou gammaprim a ensuite été obtenue par croisement des animaux hétérozygotes.

### 3) Détection du locus IgH recombiné portant le gène Cµ1 humain (allèle gamma1-KI) et du locus IgH sauvage (allèle µ sauvage) par PCR

L'ADN génomique d'un échantillon de queue des animaux homozygotes obtenus comme précisé ci-dessus, a été analysé par PCR à l'aide des deux couples d'amorces suivants :
- couple spécifique du locus IgH murin non muté (allèle µ sauvage) :
   - **amorce UpstreamSpe I Smu** : 5' gag tac cgt tgt ctg ggt cac 3' (SEQ ID NO: 14)
   - **amorce SacI-3'Imu :** 5' gag ctc tat gat tat tgg tta ac 3' (SEQ ID NO: 15)

La réaction d'amplification a été réalisée avec une température d'hybridation de 61 °C. Cette PCR amplifie en 30 cycles un fragment de 91 paires de bases-encadrant le site *Spe I*-spécifique du locus IgH murin non muté.
- couple spécifique du locus IgH recombiné portant le gène Cγ1 humain (allèle gamma1 *knock*-*in* ou gamma1-KI)
   - amorce NeoI : 5' gca tga tct gga cga aga gca t 3' (SEQ ID NO: 16)
   - amorce Neo2 : 5' tcc cct cag aag aac tcg tca a 3' (SEQ ID NO: 17)

La réaction d'amplification a été réalisée avec une température d'hybridation de 55°C. Cette PCR amplifie en 30 cycles un fragment de 120 paires de bases spécifique du locus IgH recombiné portant le gène Cγ1 humain (mutation gamma1 *knock*-*in* ou gamma1-KI).

Les animaux de la lignée gammaprim sont systématiquement et simultanément négatifs en PCR avec les amorces spécifiques de l'allèle µ sauvage et positifs en PCR avec les amorces spécifiques de l'allèle gamma1 *knock*-*in*.

### Exemple 2 : Caractérisation phénotypique de la lignée de souris transgéniques homozygotes pour la mutation gammaprim

### 1) Dosage du taux des IgM murines et des IgG1 humaines sériques totales en néphélométrie et en ELISA

### a) néphélométrie

Les IgG1 humaines sériques ont été dosées par néphélométrie sur un automate BNII™ (BEHRING) en utilisant le kit de dosage des IgG1 (BEHRING), selon les recommandations du fournisseur.

Les dosages d'IgG1 humaines sériques ont donné des résultats totalement corrélés avec ceux du génotypage réalisé par les tests PCR :
- les animaux contrôle non mutants ont un taux nul d'immunoglobulines humaines de classe IgG1
- les animaux homozygotes γ1-KI ont toujours un taux significatif d'IgG1 humaines, ce taux variant entre 0,5 et 1 g/l dans le sérum. Par contre, les IgM murines sont indétectables dans le sérum de ces animaux.

### b) ELISA

Les résultats obtenus en néphélométrie ont été confirmés en ELISA suivant les étapes suivantes : des anticorps non-marqués anti-IgG humaines (monoclonal anti-human IgG, SIGMA) ou des anticorps non-marqués anti-IgM murines (Southern Biotechnologies Associates) dilués au 1/1000^{ième} (anti-human IgG) ou au 1/500^{ème} (anti-IgM murines) en tampon carbonate 0,1 M, pH 8,3, ont été incubées une nuit à + 4° C dans des plaques à 96 puits (Maxisorb™, NUNC; 100 microlitres/puits). Après 3 lavages avec du tampon PBS contenant 0,1 % de Tween (PBS-Tween 0,1 %), les plaques ont été saturées en présence de PBS contenant 3 % de Serum albumine bovine (BSA; 100 microlitres/puits). Après 3 lavages avec du tampon PBS-Tween 0,1 %, les sérums à tester, dilués au 1/100^{ème} et au 1/1000^{ème} en tampon PBS contenant 1 % BSA ont été ajoutés (100 microlitres/puits) et les plaques ont été incubées 3 heures à 37 °C. Après 3 lavages avec du tampon PBS-Tween 0,1 %, un antisérum anti-IgG humaines marqué à la phosphatase alcaline (monoclonal anti-human IgG-AP, SIGMA) ou un sérum anti-IgM murines marqué à la phosphatase alcaline (Biosys) dilués au 1/1000^{ème} dans du PBS-Tween 0,1 % (100 microlitres/puits) ont été ajoutés et les plaques ont été incubées 3 heures à 37 °C. Après 3 lavages avec du tampon PBS-Tween 0,1 %, les IgG humaines et les IgM murines fixées ont été révélées par addition de substrat de la phosphatase alcaline (p-nitrophenylphosphate, SIGMA) à 1 mg/ml en tampon Tris 0,2M, pH 7,0. La réaction a été bloquée par addition de soude 3N (30 microlitres/puits) puis la lecture a été effectuée à l'aide d'un spectromètre, à une longueur d'onde d'absorbtion de 405 nm.

Les données quantitatives sont obtenues par extrapolation avec une gamme d'un sérum étalon (BEHRING) pour le dosage des IgG1 humaines, et d'une IgM monoclonale murine (SOUTHERN BIOTECHNOLOGIES ASSOCIATES) pour le dosage des IgM murines.

Le dosage des IgG1 sériques en ELISA montre une différence significative entre les homozygotes et les hétérozygotes ; les sérums d'homozygotes dilués au 1/500^{ième} contiennent 0,8 g/l d'IgG1 humaines, alors que des valeurs très faibles (0,03 g/l) sont observées pour les sérums d'hétérozygotes même à la dilution la plus faible (au 1/100^{ème}). A l'inverse lorsque les IgM murines sont dosées en ELISA, on observe un taux normal d'IgM murines (de l'ordre de 1 g/l) chez les souris contrôles « non mutantes » de même que chez les animaux hétérozygotes pour la mutation gammaprim. Par contre, le taux des IgM murines est nul chez les animaux homozygotes gammaprim.

### 2) Analyse de l'expression d'un récepteur membranaire de la classe des IgG humaines à la surface des lymphocytes spléniques de la lignée gammaprim

Les animaux homozygotes porteurs de la mutation γ1-KI ont été phénotypés en cytométrie de flux, par double marquage à l'aide d'anticorps spécifiques d'IgG1 humaine marqués à Alexa633 ou d'IgM murines marqués à la fluorescéine, et d'anticorps spécifiques des cellules B (anticorps anti-CD19) marqués à la phycoérythrine. De manière plus précise :
- Préparation des cellules lymphoïdes : la rate, organe lymphoïde secondaire a été choisie pour cet expérience. Cet organe a été prélevé chez différents animaux mutants homozygotes γ1-KI puis dilacérés en tampon versène (Invitrogen), et filtrés sur tamis (40 microns) afin d'obtenir une suspension de cellules individuelles débarrassée des agrégats cellulaires. Les cellules spléniques ont alors été centrifugées et soumises à une étape supplémentaire de choc osmotique afin de lyser les globules rouges par remise en suspension du culot cellulaire dans 1 ml d'eau distillée. Les cellules des échantillons ont ensuite été immédiatement remises en suspension dans du milieu complet (RPMI + 10 % sérum de veau foetal), comptées et conservées sur de la glace.
- Marquage à l'aide d'anticorps fluorescents : 10⁵ cellules ont été incubées pendant 30 minutes à 4° C avec une dilution au 1/100^{ème} d'un anticorps anti-IgM de souris marqué à l'isothiocyanante de fluorescéine (Southern Biotechnologies), d'un anticorps anti-IgG humaines couplé à Alexa 633 et d'un anticorps spécifique des cellules B (anticorps anti-CD19) marqué à la phycoérythrine. Les cellules ont ensuite été lavées dans 5 ml de PBS puis le surnageant a été décanté et les cellules ont été remises en suspension dans 100 microlitres de PBS, 0,5 % BSA, 0,1 mM EDTA.
- Analyse en cytofluorimétrie : les cellules marquées ont été analysées à l'aide d'un cytomètre de flux (COULTER XL™).

Les résultats de la cytométrie de flux sont en accord avec ceux du dosage des immunoglobulines sériques. Chez les animaux homozygotes de la lignée gamma1-KI, on ne détecte aucune expression d'IgM murines. Pourtant en l'absence d'expression d'IgM, un compartiment de cellules B périphériques CD19+ est capable de se différencier chez ces animaux et représente 6 % des lymphocytes spléniques. Ces cellules B périphériques expriment pour 40 % d'entre elles une immunoglobuline membranaire de la classe des IgG1 humaines (figure 5).

### 3) Production d'anticorps spécifiques par la lignée gammaprim

### a) Immunisation

Les animaux ont été immunisés une première fois par injection intra-péritonéale de 20 microgrammes d'antigène p24 (protéine de la nucléocapside du VIH-1) diluée dans 200 microlitres de sérum physiologique et mise en émulsion avec 200 microlitres d'adjuvant complet de Freund (SIGMA). Toutes les 2 semaines, les animaux ont subi un rappel vaccinal par injection intra-péritonéale de 20 microgrammes d'antigène 24 diluée dans 200 microlitres de sérum physiologique et mise en émulsion avec 200 microlitres d'adjuvant incomplet de Freund (SIGMA).

### b) Dosage des anticorps spécifiques de l'antigène p24

La présence d'anticorps spécifiques de l'antigène p24 a été recherchée par ELISA, 2 semaines, puis 4 semaines après la deuxième injection de l'antigène suivant la technique suivante :
- adsorption de l'antigène p24 (1 microgramme/ml en tampon carbonate 0,1 M pH 8,3) sur des plaques 96 puits (Maxisorb, NUNC; 100 microlitres/puits) par incubation une nuit à 4 °C,
- 3 lavages en PBS-Tween 0,1%,
- saturation des plaques en milieu complet (PBS, 3 % BSA; 100 microlitres/puits),
   3 lavages en PBS-Tween 0,1 %,
- distribution des sérums à tester dilés au 1/100^{ème} et au 1/200^{ème} en tampon (PBS, 1 % BSA; 100 microlitres/puits),
- incubation 3 heures à 37 °C,
- 3 lavages en PBS-Tween 0,1 %,
- distribution d'un antisérum anti-IgG humaines marqué à la phosphatase alcaline (monoclonal anti-human IgG-AP, SIGMA) dilué au 1/1000^{ème} (100 microlitres/puits)
- 3 lavages en PBS-Tween 0,1 %,
- révélation de la réaction enzymatique par addition de p-nitrophenylphosphate en tampon TRIS 0,2M (SIGMA),
- blocage de la réaction par addition de soude 3N (30 microlitres/puits),
- lecture en spectrométrie à une longueur d'onde d'absortion de 405 nm,
- le taux des anticorps IgG anti-p24 est exprimé en unités arbitraires (UA), établies pour la moyenne des sérums dilués au 1/100^{ème} et 1/200^{ème} en fonction du rapport Densité optique sérum testé / Densité optique du sérum témoin.

La présence d'anticorps spécifiques de l'antigène p24 a été détectée par ELISA à partir de J26 (après 2 immunisations - 100 UA), et est fortement démontrée à J47 (3 semaines après la deuxième injection de l'antigène - 450 UA; figure 6). En comparaison, il a été vérifié qu'en l'absence d'immunisation des animaux, le taux des anticorps IgG1 anti-p24 détectés restait inférieur à 5 unités.

### Exemple 3: Obtention et caractérisation de la lignée double-transgénique gammaprim/kappa exprimant une chaîne lourde gamma1 chimérique et une chaîne légère kappa d'immunoglobulines humaines

Le croisement des lignées κARN exprimant une chaîne légère kappa d'immunoglobuline humaine, telle que décrite dans la Demande Internationale PCT WO 2005/047333 et gamma1-KI décrite aux exemples précédents, génère des souris doubles transgéniques gammaprim/kappa.

Pour ce faire, les animaux homozygotes pour la mutation gamma1-KI et homozygotes pour le transgène κ ARN ont été croisés entre eux. En première génération (F1) après ce croisement, tous les animaux obtenus sont hétérozygotes pour la mutation gamma1-KI et hétérozygotes pour le transgène κ ARN. Ces animaux F1 ont donc été à nouveau croisés : à la génération suivante (F2) les lois de la génétique Mendélienne permettent d'obtenir 1 animal sur 4 homozygote pour la mutation gamma1-KI et un animal sur 4 homozygote pour le transgène κ ARN. Parmi ces animaux F2, un animal sur 16 a donc pu être sélectionné comme portant à la fois la mutation gamma1-KI à l'état homozygote et comme portant le transgène κ ARN à l'état homozygote. Ces animaux sont les fondateurs de la lignée gammaprim/kappa et ils transmettent de façon stable à leur descendance les gènes permettant simultanément la production d'une chaîne lourde gamma1 humanisée en remplacement de la production d'IgM murines ainsi que la production et la diversification par hypermutation d'une chaîne κ humaine.

Cette lignée de souris double-transgéniques est dénommé lignée gammaprim/kappa

### a) présence du transgene κ

La transmission du transgène κ ARN au cours des croisements de souris transgéniques a été suivie par Southern Blot à l'aide d'une sonde spécifique de la région Cκ humaine (fragment *EcoR*I-*EcoR*I de 2,5 kb incluant la totalité de l'exon Cκ humain).

L'expression du transgène κ chez les animaux mutants a été détectée par dosage en ELISA de chaînes kappa libres humaines éliminées dans les urines des animaux. De manière plus, précise : des plaques à 96 puits (Maxisorb®, NUNC) ont été incubées une nuit à + 4° C en présence d' un anticorps non marqué anti-κ humain (Kallestad) dilué au 1/1000^{ème} en tampon carbonate 0,1 M pH 8,3 (100 microlitres/puits). Après 3 lavages avec du tampon PBS contenant 0,1 % de Tween (PBS-Tween 0,1 %), les plaques ont été saturées en présence de PBS contenant 10 % sérum de veau foetal (100 microlitres/puits). Après 3 lavages avec du tampon PBS-Tween 0,1 %, les échantillons d'urine à tester, dilués au 1/100^{ème} et au 1/500^{ème} en tampon PBS contenant 10 % de sérum de veau foetal ont été ajoutés (100 microlitres/puits) et les plaques ont été incubées 3 heures à 37 °C. Après 3 lavages avec du tampon PBS-Tween 0,1 %, un antisérum anti-κ humain marqué à la phosphatase alcaline (SIGMA) dilué au 1/1000^{ème} dans du PBS-Tween 0,1% (100 microlitres/puits) a été ajouté et les plaques ont été incubées 1 heure à 37°C. Après 3 lavages avec du tampon PBS-Tween 0,1 %, les chaînes légères kappa humaines fixées ont été révélées par addition de substrat de la phosphatase alcaline (p-nitrophényl-phosphate, SIGMA) à 1 mg/ml en tampon Tris 0,2M, pH 7,0. La réaction a été bloquée par addition de soude 0,5 N (50 microlitres/puits) puis la lecture a été effectuée par spectrométrie à une longueur d'onde d'absorbtion de 405 nm.

Alternativement, l'expression du transgène kappa humain a été analysée en cytométrie de flux, par double-marquage à l'aide d'un anticorps anti-kappa murin (marqué à la phycoérythrine) en conjonction avec un anticorps anti-kappa humain (marqué à l'isothiocyanate de fluorescéine), selon le protocole décrit à l'exemple 2. Les résultats montrent que la présence du trànsgène κ ARN entraîne un phénomène important d'exclusion allélique, tel que parmi les lymphocytes périphériques plus de 50 % expriment la chaîne légère humaine et ne réarrangent donc pas les gènes de chaîne légères murines pour exprimer une chaîne légère murine.

### b) homozygotie pour la mutation gamma1-KI

Le premier élément simple indiquant l'homozygotie gamma1-KI est la présence d'un taux élevé d'IgG1 humaines dans le sérum des animaux. En outre l'homozygotie a été confirmée en PCR par la positivité de la « PCR gamma1-KI» associée à la négativité de la «PCR allèle µ sauvage ». Enfin, après sacrifice des animaux, l'analyse en cytométrie de flux a permis de montrer sur les lymphocytes de la rate que la totalité des lymphocytes B (CD19+) expriment des IgG1 humaines membranaire alors qu'en parallèle aucune cellule B n'exprime d'IgM murine.

### c) Vérification de la présence simultanée de la mutation alpha1-KI et du transgène κ ARN chez les animaux gammaprim/kappa et leur descendance.

Les animaux double-transgéniques gammaprim/kappa ont été caractérisés comme ceux répondant simultanément aux deux spécificités décrites ci-dessus : présence du transgène κ ARN à l'état homozygote et homozygotie pour la mutation gamma1-KI. De plus, ces animaux se reproduisent en préservant ces deux spécificités et le phénotype de leur descendance possède les propriétés suivantes, simultanément et de façon stable :
- la production d'IgG1 humanisée à un taux conséquent (facilement vérifiable par ELISA ou néphélométrie sur un simple prélèvement de sang réalisé chez les animaux vivants au niveau du sinus rétro-orbitaire)
- la production de chaîne légère κ humaine (facilement vérifiable par ELISA sur un simple prélèvement d'urines réalisé chez les animaux vivants).

Le répertoire de réponse aux antigènes de ces souris est attendu comme subnormal puisqu'on sait que c'est essentiellement le domaine VH de la chaîne lourde qui contribue à la formation du site anticorps (or la chaîne lourde transgénique γ1 humaine bénéficie d'un répertoire totalement diversifié puisque correspondant au répertoire normal généré par les réarrangements des segments VH, D et JH du locus IgH murin.). Ces souris sont capables de produire des anticorps de forte affinité en réponse secondaire, ce qui résulte du fait que leurs lymphocytes B peuvent recruter le phénomène d'hypermutation somatique à la fois au niveau du gène de chaîne lourde et du transgène de chaîne légère κ *ARN*.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Souris transgénique, **caractérisée en ce qu'**elle comprend un locus IgH modifié par remplacement de la séquence de commutation Sµ par tout ou partie d'un transgène constitué par le gène Cγ d'une immunoglobuline humaine de classe G, incluant au moins l'exon codant pour le domaine CH3 et les exons de membrane m1 et m2 et **en ce qu'**elle produit des anticorps qui sont majoritairement des IgG chimériques dont les domaines constants des chaînes lourdes sont d'origine humaine.

2. Souris transgénique selon la revendication 1, **caractérisée en ce qu'**elle est homozygote pour ledit locus IgH modifié.

3. Souris transgénique selon la revendication 1 ou la revendication 2, **caractérisée en ce que** ledit locus IgH est modifié par remplacement de la séquence de commutation Sµ par la totalité du gène Cγ.

4. Souris transgénique selon l'une quelconque des revendications 1 à 3, **caractérisée en ce que** ledit gène Cγ est le gène Cγ1.

5. Souris transgénique selon l'une quelconque des revendications 1 à 4, **caractérisée en ce qu'**elle comprend un autre transgène codant pour une chaîne légère d'immunoglobuline humaine.

6. Souris transgénique selon la revendication 5, **caractérisée en ce que** le dit transgène est un gène kappa humain.

7. Souris transgénique selon la revendication 6, **caractérisée en ce que** ledit gène kappa humain présente la séquence SEQ ID NO : 9.

8. Souris transgénique selon l'une quelconque des revendications 5 à 7, **caractérisée en ce qu'**elle est homozygote ou hétérozygote pour ledit transgène.

9. Souris transgénique selon l'une quelconque des revendications 5 à 8, **caractérisée en ce qu'**elle possède un locus endogène de la chaîne kappa inactivé.

10. Vecteur de ciblage de la recombinaison homologue, **caractérisé en ce qu'**il comprend le gène Cγ d'une immunoglobuline humaine de classe G ou un segment de ce gène incluant au moins l'exon codant pour le domaine CH3 et les exons de membrane m1 et m2 tel que défini à l'une quelconque des revendications 1, 3 ou 4, flanqué de fragments de séquences du locus IgH murin qui sont adjacents à la séquence Sµ.

11. Vecteur de ciblage selon la revendication 10, **caractérisé en ce que** lesdits fragments de séquences flanquantes qui sont adjacentes à la séquence Sµ sont les séquences murines SEQ ID NO : 11 et 12, respectivement en 5' et en 3'.

12. Cellule embryonnaire de souris, modifiée par un vecteur de ciblage selon la revendication 10 ou la revendication 11.

13. Anticorps chimérique de classe IgG susceptible d'être obtenu à partir des lymphocytes B d'une souris transgénique selon l'une quelconque des revendications 5 à 9 ou fragment Fab, Fab'2 ou Fc dudit anticorps, **caractérisé en ce que** ledit anticorps comprend une chaîne lourde chimérique dont les domaines constants sont d'origine humaine et les domaines variables sont d'origine murine et des chaînes légères humaines dont les domaines variables sont codés par Vκ1-Jκ5.

14. Réactif de diagnostic, **caractérisé en ce qu'**il comprend un anticorps chimérique de classe IgG ou un fragment de cet anticorps selon la revendication 13.

15. Composition pharmaceutique, **caractérisée en ce qu'**elle comprend au moins un anticorps chimérique de classe IgG ou un fragment de cet anticorps selon la revendication 13.

## Patentansprüche

1. Transgene Maus, **dadurch gekennzeichnet, dass** sie einen IgH-Locus umfasst, der durch Ersatz der Kommutationssequenz Sµ durch die Gesamtheit oder einen Teil eines Transgens, das aus dem Gen Cγ des humanen Immunglobulins der Klasse G besteht, einschließlich wenigstens des Exons, das für die CH3-Domäne codiert, und der Membranexons m1 und m2, und dadurch, dass sie Antikörper produziert, die hauptsächlich chimäre IgG sind, deren konstante Domänen der schweren Ketten humaner Herkunft sind.

2. Transgene Maus gemäß Anspruch 1, **dadurch gekennzeichnet, dass** sie bezüglich des modifizierten IgH-Locus homozygot ist.

3. Transgene Maus gemäß Anspruch 1 oder Anspruch 2, **dadurch gekennzeichnet, dass** der IgH-Locus durch Ersatz der Kommutationssequenz Sµ durch die Gesamtheit des Gens Cγ modifiziert ist.

4. Transgene Maus gemäß einem der Ansprüche 1 bis 3, **dadurch gekennzeichnet, dass** das Gen Cγ das Gen Cγ1 ist.

5. Transgene Maus gemäß einem der Ansprüche 1 bis 4, **dadurch gekennzeichnet, dass** sie ein anderes Transgen umfasst, das für eine leichte Kette von humanem Immunglobulin codiert.

6. Transgene Maus gemäß Anspruch 5, **dadurch gekennzeichnet, dass** das Transgen ein humanes kappa-Gen ist.

7. Transgene Maus gemäß Anspruch 6, **dadurch gekennzeichnet, dass** das humane kappa-Gen die Sequenz SEQ ID NO:9 aufweist.

8. Transgene Maus gemäß einem der Ansprüche 5 bis 7, **dadurch gekennzeichnet, dass** sie bezüglich des Transgens homozygot oder heterozygot ist.

9. Transgene Maus gemäß einem der Ansprüche 5 bis 8, **dadurch gekennzeichnet, dass** sie einen inaktivierten endogenen Locus der kappa-Kette besitzt.

10. Targeting-Vektor für die homologe Rekombination, **dadurch gekennzeichnet, dass** er das Gen Cγ eines humanen Immunglobulins der Klasse G oder ein Segment dieses Gens, einschließlich wenigstens des Exons, das für die CH3-Domäne codiert, und der Membranexons m1 und m2, umfasst, wie es in einem der Ansprüche 1, 3 oder 4 definiert ist, flankiert von Fragmenten der Sequenzen des murinen IgH-Locus, die der Sequenz Sµ benachbart sind.

11. Targeting-Vektor gemäß Anspruch 10, **dadurch gekennzeichnet, dass** die flankierenden Sequenzfragmente, die der Sequenz Sµ benachbart sind, die murinen Sequenzen SEQ ID NO:11 und 12, in 5' bzw. in 3' sind.

12. Embryonale Mauszelle, die durch einen Targeting-Vektor gemäß Anspruch 10 oder Anspruch 11 modifiziert ist.

13. Chimärer Antikörper der IgG-Klasse, der ausgehend von B-Lymphozyten einer transgenen Maus gemäß einem der Ansprüche 5 bis 9 erhalten werden kann, oder Fab-, Fab'2- oder Fc-Fragment des Antikörpers, **dadurch gekennzeichnet, dass** der Antikörper eine chimäre schwere Kette, deren konstante Domänen humanen Ursprungs sind, und deren variable Domänen murinen Ursprungs sind, und humane leichte Ketten, deren variable Domänen durch Vκ1-Jκ5 codiert werden, umfasst.

14. Diagnosereagens, **dadurch gekennzeichnet, dass** es einen chimären Antikörper der Klasse IgG oder ein Fragment dieses Antikörpers gemäß Anspruch 13 umfasst.

15. Pharmazeutische Zusammensetzung, **dadurch gekennzeichnet, dass** sie wenigstens einen chimären Antikörper der Klasse IgG oder ein Fragment dieses Antikörpers gemäß Anspruch 13 umfasst.

## Claims

1. Transgenic mouse, **characterised in that** it comprises an IgH locus which is modified by replacement of the switch sequence Sµ by all or a portion of a transgene comprising the Cγ gene of a class G human immunoglobulin, including at least the exon coding for the CH3 domain and the membrane exons m1 and m2 and **in that** it produces antibodies which are primarily chimeric IgG whose heavy-chain constant domains are of human origin.

2. Transgenic mouse according to claim 1, **characterised in that** it is homozygous for the modified IgH locus.

3. Transgenic mouse according to claim 1 or claim 2, **characterised in that** the IgH locus is modified by replacement of the switch sequence Sµ by the whole of the Cγ gene.

4. Transgenic mouse according to any one of claims 1 to 3, **characterised in that** the Cγ gene is the gene Cγ1.

5. Transgenic mouse according to any one of claims 1 to 4, **characterised in that** it comprises another transgene coding for a light chain of human immunoglobulin.

6. Transgenic mouse according to claim 5, **characterised in that** the transgene is a human kappa gene.

7. Transgenic mouse according to claim 6, **characterised in that** the human kappa gene has the sequence SEQ ID NO: 9.

8. Transgenic mouse according to any one of claims 5 to 7, **characterised in that** it is homozygous or heterozygous for the transgene.

9. Transgenic mouse according to any one of claims 5 to 8, **characterised in that** it has an inactivated endogenous locus of the kappa chain.

10. Targeting vector for homologous recombination, **characterised in that** it comprises the Cγ gene of a class G human immunoglobulin, or a segment of this gene which includes at least the exon coding for the CH3 domain and the membrane exons m1 and m2 as defined in any one of claims 1, 3 or 4, flanked by fragments of sequences of the murine IgH locus which are adjacent to the Sµ sequence.

11. Targeting vector according to claim 10, **characterised in that** the fragments of flanking sequences which are adjacent to the Sµ sequence are the murine sequences SEQ ID NO: 11 and 12, at 5' and 3', respectively.

12. Embryonic mouse cell, modified by a targeting vector according to claim 10 or claim 11.

13. Chimeric antibody of the class IgG which is capable of being obtained from the B lymphocytes of a transgenic mouse according to any one of claims 5 to 9 or fragment Fab, Fab'2 or Fc of the antibody, **characterised in that** the antibody comprises a heavy chimeric chain, of which the constant regions are of human origin and the variable regions are of murine origin, and light human chains, of which the variable regions are encoded by Vκ1 - Jκ5.

14. Diagnostic reagent, **characterised in that** it comprises a chimeric antibody of the class IgG or a fragment of this antibody according to claim 13.

15. Pharmaceutical composition, **characterised in that** it comprises at least one chimeric antibody of the class IgG or a fragment of this antibody according to claim 13.


### SEQUENCE LISTING

<110> UNIVERSITE DE LIMOGES
   CENTRE NATIONAL DE LA RECHERCHE SCIENTIFIQUE
   COGNE,Michel
   RAYNAL,Sophie
   COGNE,Nadine
   PINAUD,Eric
<120> Mammifère non-humain transgénique pour la région constante de la chaîne lourde des immunoglobulines humaines de classe G et ses applications
<130> F2052PCT1
<160> 17
<170> PatentIn version 3.3
<210> 1
   <211> 24
   <212> DNA
   <213> Artificial
<220> primer
   <223> GamlCH1-5'F
<400> 1
   caatcgatgc ccgtgagccc agac 24
<210> 2
   <211> 20
   <212> DNA
   <213> artificial
<220> primer
   <223> Gam1 CH1-3'R
<400> 2
   gcttccagca gagacaccct 20
<210> 3
   <211> 18
   <212> DNA
   <213> Artificial
<220> primer
   <223> Gam1 CH2-5'F
<400> 3
   cagctcggac accttctc 18
<210> 4
   <211> 18
   <212> DNA
   <213> Artificial
<220> primer
   <223> Gam1 CH2-3'R
<400> 4
   ccggcctctg tccatgtg 18
<210> 5
   <211> 18
   <212> DNA
   <213> Artificial
<220> primer
   <223> Gam1 CH3-5'F
<400> 5
   cacatggaca gaggccgg 18
<210> 6
   <211> 18
   <212> DNA
   <213> Artificial
<220> primer
   <223> Gam1 CH3-3'R
<400> 6
   ctgacccgtg gaaagaac 18
<210> 7
   <211> 18
   <212> DNA
   <213> Artificial
<220> primer
   <223> Gam1 mbexon-5'F
<400> 7
   agctgacctc aggacatt 18
<210> 8
   <211> 28
   <212> DNA
   <213> Artificial
<220> primer
   <223> Gam1 mbexon-5'F
<400> 8
   aaatcgatgc tcccatcacg aagtacaa 28
<210> 9
   <211> 405
   <212> DNA
   <213> Homo sapiens
<400> 9
   atggacatga gggtccctgc tcagctcctg gggctcctgc tgctctggct ctcaggtgcc 60
   agatgtgaca tccagatgac ccagtctcca tcttccctgt ctgcatctgt aggagacgga 120
   gtcaccctca cttgccaggc gagtcaggac attagcgact atttaaattg gtatcagcag 180
   aaagtagggg aagcccctaa gctcctgatg tacgatgcat catacttgga aacaggggtc 240
   ccattaagat tcagtggaag tggatctggg acaaattata gtttcaccat cagcagcctg 300
   cagcctgaag attttgcaac atattactgt caacagtatt ctaatctccc tttcaccttc 360
   ggccaaggga cacgactgga gattaaacga actgtggcac catct 405
<210> 10
   <211> 135
   <212> PRT
   <213> Homo sapiens
<400> 10
   Met Asp Met Arg Val Pro Ala Gln Leu Leu Gly Leu Leu Leu Leu Trp
   1               5                   10                  15
   Leu Ser Gly Ala Arg Cys Asp Ile Gln Met Thr Gln Ser Pro Ser Ser
               20                  25                  30
   Leu Ser Ala Ser Val Gly Asp Gly Val Thr Leu Thr Cys Gln Ala Ser
           35                  40                  45
   Gln Asp Ile Ser Asp Tyr Leu Asn Trp Tyr Gln Gln Lys Val Gly Glu
       50                  55                  60
   Ala Pro Lys Leu Leu Met Tyr Asp Ala Ser Tyr Leu Glu Thr Gly Val
   65                  70                  75                  80
   Pro Leu Arg Phe Ser Gly Ser Gly Ser Gly Thr Asn Tyr Ser Phe Thr
                   85                  90                  95
   Ile Ser Ser Leu Gln Pro Glu Asp Phe Ala Thr Tyr Tyr Cys Gln Gln
               100                 105                 110
   Tyr Ser Asn Leu Pro Phe Thr Phe Gly Gln Gly Thr Arg Leu Glu Ile
           115                 120                 125
   Lys Arg Thr Val Ala Pro Ser
       130                 135
<210> 11
   <211> 5161
   <212> DNA
   <213> Mus musculus
<400> 11
<210> 12
   <211> 4932
   <212> DNA
   <213> Mus musculus
<400> 12
   tagcagggtg tagagggatc tcctgtctga caggaggcaa gaagacagat tcttacccct      60
   ccatttctct tttatccctc tctggtcctc agagagtcag tccttcccaa atgtcttccc     120
   cctcgtctcc tgcgagagcc ccctgtctga taagaatctg gtggccatgg gctgcctggc     180
   ccgggacttc ctgcccagca ccatttcctt cacctggaac taccagaaca acactgaagt     240
   catccagggt atcagaacct tcccaacact gaggacaggg ggcaagtacc tagccacctc     300
   gcaggtgttg ctgtctccca agagcatcct tgaaggttca gatgaatacc tggtatgcaa     360
   aatccactac ggaggcaaaa acaaagatct gcatgtgccc attccaggta agaaccaaac     420
   cctcccagca ggggtgccca ggcccaggca tggcccagag ggagcagcgg ggtggggctt     480
   aggccaagct gagctcacac cttgaccttt cattccagct gtcgcagaga tgaaccccaa     540
   tgtaaatgtg ttcgtcccac cacgggatgg cttctctggc cctgcaccac gcaagtctaa     600
   actcatctgc gaggccacga acttcactcc aaaaccgatc acagtatcct ggctaaagga     660
   tgggaagctc gtggaatctg gcttcaccac agatccggtg accatcgaga acaaaggatc     720
   cacaccccaa acctacaagg tcataagcac acttaccatc tctgaaatcg actggctgaa     780
   cctgaatgtg tacacctgcc gtgtggatca caggggtctc accttcttga agaacgtgtc     840
   ctccacatgt gctgccagtg agtggcctgg gctaagccca atgcctagcc ctcccagatt     900
   agggaagtcc tcctacaatt atggccaatg ccacccagac atggtcattt gctccttgaa     960
   ctttggctcc ccagagtggc caaggacaag aatgagcaat aggcagtaga ggggtgagaa    1020
   tcagctggaa ggaccagcat cttcccttaa gtaggtttgg gggatggaga ctaagctttt    1080
   ttccaacttc acaactagat atgtcataac ctgacacagt gttctcttga ctgcaggtcc    1140
   ctccacagac atcctaacct tcaccatccc cccctccttt gccgacatct tcctcagcaa    1200
   gtccgctaac ctgacctgtc tggtctcaaa cctggcaacc tatgaaaccc tgaatatctc    1260
   ctgggcttct caaagtggtg aaccactgga aaccaaaatt aaaatcatgg aaagccatcc    1320
   caatggcacc ttcagtgcta agggtgtggc tagtgtttgt gtggaagact ggaataacag    1380
   gaaggaattt gtgtgtactg tgactcacag ggatctgcct tcaccacaga agaaattcat    1440
   ctcaaaaccc aatggtaggt atcccccctt cccttcccct ccaattgcag gacccttcct   1500
   gtacctcata gggagggcag gtcctcttcc accctatcct cactactgtc ttcatttaca   1560
   gaggtgcaca aacatccacc tgctgtgtac ctgctgccac cagctcgtga gcaactgaac   1620
   ctgagggagt cagccacagt cacctgcctg gtgaagggct tctctcctgc agacatcagt   1680
   gtgcagtggc ttcagagagg gcaactcttg ccccaagaga agtatgtgac cagtgccccg   1740
   atgccagagc ctggggcccc aggcttctac tttacccaca gcatcctgac tgtgacagag   1800
   gaggaatgga actccggaga gacctatacc tgtgttgtag gccacgaggc cctgccacac   1860
   ctggtgaccg agaggaccgt ggacaagtcc actggtaaac ccacactgta caatgtctcc   1920
   ctgatcatgt ctgacacagg cggcacctgc tattgaccat gctagcgctc aaccaggcag   1980
   gccctgggtg tccagttgct ctgtgtatgc aaactaacca tgtcagagtg agatgttgca   2040
   ttttataaaa attagaaata aaaaaaatcc attcaaacgt cactggtttt gattatacaa   2100
   tgctcatgcc tgctgagaca gttgtgtttt gcttgctctg cacacaccct gcatacttgc   2160
   ctccaccctg gcccttcctc taccttgcca gtttcctcct tgtgtgtgaa ctcagtcagg   2220
   cttacaacag acagagtatg aacatgcgat tcctccagct acttctagat atatggctga   2280
   aagcttgcct aacctggtgc aggcagcatt caggcacata tatagacaca catgcattta   2340
   tacatagata tataggtaca catgtgtaga cacatacatg aatgtgtatt catggacaca   2400
   cagacaaagg tacacatata tacacatgag ttcatgcgca cacacatgca tggacactta   2460
   caaacgcctt cagagacaaa taggcataga cacacaacca ctcacagaaa cagataccaa   2520
   tatgcatggt cctgtgtaca cagaaacaga ctataggcaa atatacacaa ataaactata   2580
   tagatacaaa gatatgcata tacacacatg tacagaaaca tcttcacatg tgtacactaa   2640
   catgtgaaca ggtatagcac acagatacac ctggactctg accagggctg taatctccaa   2700
   ggctcacggc tcagagagcc tacactaggc tgggtcactg atactcctca ggagcccact   2760
   ctatgattgg gagagataac cccaggtaca aagtatgcct atctgtctca acaccatggg   2820
   gcagaagata ctccactaac cacccatgac agaaagttag ccttggctgt gtctccatta   2880
   atagaacacc tcagaagacc aatgtgaaat tgcctaaccc actcacaccc accctgatct   2940
   ccagttcaaa atgcagaaaa cataatgcag ttgtccaaaa gatgccccaa ccacacacac   3000
   acacacacac acacacacac acacacacac acacacacac acacacacac accatcaagg   3060
   agcctctgta aggagtcacc acccaataac actgcctctt tgggctcata tcctggacat   3120
   tcttcatatt catatccatt tggggcctag gctttagata tccccaaggg ctcatcttta   3180
   cagggatcag agatcccaat aaatgccctg gtcccacagc ctccctcagg tatctgtctg   3240
   tttatctctt ggtaccaaga cccaacattg ctggcagggg taggacaagc aacgcacggg   3300
   aactctgatc aaagaaagtc atgagatgcc tgagtccttc aggaagtaag gagggacaac   3360
   ctctggtatc cctgttctta ttgctaaagc ccaagagaca gggagacctg ctctaaattc   3420
   tcagtctaaa cagcaccgat ggcaccacct gctcagggaa agtccagagc acaccaatat   3480
   cattttgcca cagttcctga gtctgccttt acccaggtcc atacattgca tctgtcttgc    3540
   ttgctctgct gccccagggc tcctggaaca aaggctccaa attagtgtgt cctacagctt    3600
   ggcctgttct gtgcctccgt ctagcttgag ctattagggg accagtcaat actcgctaag    3660
   attctccaga accatcaggg caccccaacc cttatgcaaa tgctcagtca ccccaagact    3720
   tggcttgacc ctccctctct gtgtcccttc atagaggggg aggtgaatgc tgaggaggaa    3780
   ggctttgaga acctgtggac cactgcctcc accttcatcg tcctcttcct cctgagcctc    3840
   ttctacagca ccaccgtcac cctgttcaag gtagtgtggt tgtggggctg aggacacagg    3900
   gctgggacag ggagtcacca gtcctcactg cctctacctc tactccctac aagtggacag    3960
   caattcacac tgtctctgtc acctgcaggt gaaatgactc tcagcatgga aggacagcag    4020
   agaccaagag atcctcccac agggacacta cctctgggcc tgggatacct gactgtatga    4080
   ctagtaaact tattcttacg tctttcctgt gttgccctcc agcttttatc tctgagatgg    4140
   tcttctttct agactgacca aagacttttt gtcaacttgt acaatctgaa gcaatgtctg    4200
   gcccacagac agctgagctg taaacaaatg tcacatggaa ataaatactt tatcttgtga    4260
   actcacttta ttgtgaagga atttgttttg tttttcaaac ctttcctgcg gtgttgacag    4320
   cccaaggatt atctgaatag agcttaggaa ctggaaatgg aacagtgcag tctgatggta    4380
   cttaagggag aaagagggaa aggaggtgtg gaagaagaaa aaagagaagc agagggggag    4440
   gggagaaggg agagggagag ggagagggag agggagaggg agagggagag ggagagagag    4500
   agagagagag agagagagag agagagagag agagagcatg cactctaaca gcaaagtaca    4560
   acacaggcag ccaatggata gcactctggt tatctaccct gatggaagaa gggaagtagg    4620
   gcagagaaaa ttccaggcct aatctcccaa aagcaacaga acctggaaac tagcctctag    4680
   ccttaggtct ctgctctgtc cccagcccac catcttgggc tggtgttgct tcaagctagt    4740
   aatttaggtc ttatcccaaa gctttgtggt atgtgggtgt gcctttgggg agttggctga    4800
   gattttgaag atgtttgtac ctctcccaca acatgacaag ccctaggggt tagtcaataa    4860
   ctcaaattct ctgtctatga caactgctgt atgactatat gaagaaatgg gataaagatg    4920
   ctatagtcac tc                                                        4932
<210> 13
   <211> 6864
   <212> DNA
   <213> Homo sapiens
<400> 13
   atgcccgtga gcccagacac tggacgctga acctcgcgga cagttaagaa cccaggggcc      60
   tctgcgccct gggcccagct ctgtcccaca ccgcggtcac atggcaccac ctctcttgca     120
   gcctccacca agggcccatc ggtcttcccc ctggcaccct cctccaagag cacctctggg     180
   ggcacagcag ccctgggctg cctggtcaag gactacttcc ccgaaccggt gacggtgtcg     240
   tggaactcag gcgccctgac cagcggcgtg cacaccttcc cggctgtcct acagtcctca     300
   ggactctact ccctcagcag cgtggtgacc gtgccctcca gcagcttggg cacccagacc     360
   tacatctgca acgtgaatca caagcccagc aacaccaagg tggacaagaa agttggtgag 420
   aggccagcac agggagggag ggtgtctgct ggaagccagg ctcagcgctc ctgcctggac 480
   gcatcccggc tatgcagccc cagtccaggg cagcaaggca ggccccgtct gcctcttcac 540
   ccggaggcct ctgcccgccc cactcatgct cagggagagg gtcttctggc tttttcccca 600
   ggctctgggc aggcacaggc taggtgcccc taacccaggc cctgcacaca aaggggcagg 660
   tgctgggctc agacctgcca agagccatat ccgggaggac cctgcccctg acctaagccc 720
   accccaaagg ccaaactctc cactccctca gctcggacac cttctctcct cccagattcc 780
   agtaactccc aatcttctct ctgcagagcc caaatcttgt gacaaaactc acacatgccc 840
   accgtgccca ggtaagccag cccaggcctc gccctccagc tcaaggcggg acaggtgccc 900
   tagagtagcc tgcatccagg gacaggcccc agccgggtgc tgacacgtcc acctccatct 960
   cttcctcagc acctgaactc ctggggggac cgtcagtctt cctcttcccc ccaaaaccca 1020
   aggacaccct catgatctcc cggacccctg aggtcacatg cgtggtggtg gacgtgagcc 1080
   acgaagaccc tgaggtcaag ttcaactggt acgtggacgg cgtggaggtg cataatgcca 1140
   agacaaagcc gcgggaggag cagtacaaca gcacgtaccg tgtggtcagc gtcctcaccg 1200
   tcctgcacca ggactggctg aatggcaagg agtacaagtg caaggtctcc aacaaagccc 1260
   tcccagcccc catcgagaaa accatctcca aagccaaagg tgggacccgt ggggtgcgag 1320
   ggccacatgg acagaggccg gctcggccca ccctctgccc tgagagtgac cgctgtacca 1380
   acctctgtcc ctacagggca gccccgagaa ccacaggtgt acaccctgcc cccatcccgg 1440
   gatgagctga ccaagaacca ggtcagcctg acctgcctgg tcaaaggctt ctatcccagc 1500
   gacatcgccg tggagtggga gagcaatggg cagccggaga acaactacaa gaccacgcct 1560
   cccgtgctgg actccgacgg ctccttcttc ctctacagca agctcaccgt ggacaagagc 1620
   aggtggcagc aggggaacgt cttctcatgc tccgtgatgc atgaggctct gcacaaccac 1680
   tacacacaga agagcctctc cctgtctccg ggtaaatgag tgccacggcc ggcaagcccc 1740
   cgctccccag gctctcgggg tcgcgcgagg atgcttggca cgtaccccgt gtacatactt 1800
   cccaggcacc cagcatggaa ataaagcacc cagcgcttcc ctgggcccct gcgagactgt 1860
   gatggttctt tccacgggtc aggccgagtc tgaggcctga gtggcatgag ggaggcagag 1920
   tgggtcccac tgtccccaca ctggcccagg ctgtgcaggt gtgcctgggc cgcctagggt 1980
   ggggctcagc caggggctgc cctcggcagg gtgggggatt tgccagcgtg gccctccctc 2040
   cagcagcagc tgccctgggc tgggccacga gaagccctag gagcccctgg ggacagacac 2100
   acagcccctg cctctgtagg agactgtcct gttctgtgag cgccctgtcc tccgacccgc 2160
   atgcccactc gggggcatgc ctagtccatg tgcgtaggga caggccctcc ctcacccatc 2220
   tacccccacg gcactaaccc ctggcagccc tgcccagcct cgcacccgca tggggacaca 2280
   accgactccg gggacatgca ctctcgggcc ctgtggagag actggtccag atgcccacac 2340
   acacactcag cccagacccg ttcaacaaac cccgcactga ggttggccgg ccacacggcc 2400
   accacacaca cacgtgcacg cctcacacac ggagcctcac ccgggcgaac cgcacagcac 2460
   ccagaccaga gcaaggtcct cgcacacgtg aacactcctc ggacacaggc ccccacgagc 2520
   cccacgcggc acctcaaggc ccacgagccg ctcggcagct tctccacatg ctgacctgct 2580
   cagacaaacc cagccctcct ctcacaaggt gcccctgcag ccgccacaca cacacagggg 2640
   atcacacacc acgtcacgtc cctggccctg gcccacttcc cagtgccgcc cttccctgca 2700
   gctggggtca catgaggtgt gggcttcacc atcctcctgc cctctgggcc tcagggaggg 2760
   acacgggaga cggggagcgg gtcctgctga gggccaggtc gctatctagg gccgggtgtc 2820
   tggctgagcc ccggggccaa agctggtgcc cagggcgggc agctgtgggg agctgacctc 2880
   aggacattgt tggcccatcc cggccgggcc ctacatcctg ggtcctgcca cagagggaat 2940
   cacccccaga ggcccaagcc cagggggaca cagcactgac cacccccttc ctgtccagag 3000
   ctgcaactgg aggagagctg tgcggaggcg caggacgggg agctggacgg gctgtggacg 3060
   accatcacca tcttcatcac actcttcctg ttaagcgtgt gctacagtgc caccgtcacc 3120
   ttcttcaagg tcggccgcac gttgtcccca gctgtccttg acattgtccc ccatgctgtc 3180
   acaaactgtc tctgacactg tcccacaggc tgtccccacc tgtccctgac gctgtccccc 3240
   atgctctcac aaactgtccc tgacattgtc cccaatgctg cccccacctg tccaacagtg 3300
   tcccccaggc tctccccaca tgtccccgac actgtccccc atgctgtccc catctgtccc 3360
   caacactgtc ccccaccctg tccccctttg tccccaacac tgtcccccac agtttccacc 3420
   tgtccctgac actgtccccc atgctttccc cacctgtccc tgacaccatc ccccactctg 3480
   tcccctatag ttcctggccc tgtcccccac gctgtcccct acagtacctg gcactgtccc 3540
   ccatgctgtc ccctcctgta tgaaaccctg tcccacatgc tgtccccacc tgtccgtgac 3600
   aatatccccc acactgtccc cacctgtccc cgacactctc ctccacgttg ttcttaccta 3660
   aacccgacac tttcctccat gctgtcccca cccatctccg acactgtacc ccacgttgtc 3720
   cccacctgtc ctcaacactg tcccccatgc tgtccccacc tgtccccaac actctcctcc 3780
   atgctgtccc cacctgtccc tgatattgtc ccccatgcag tctccacctg tccccaatgc 3840
   tgtcccccag gctgtaccta ccagtacaac actgtccccc atgctgtccc cacctgtccc 3900
   tgacactgtc ccccacgctg tcccctcctg tccccgacac tgtcccccac actgtcccca 3960
   cctgtcccca acactatcct ccatgctgtc ccctcctgtc cccacctgtc ccctacactg 4020
   tcccccatgc tgtccccacc agtccccaaa actttcctcc acactgtccc cacctgtccc 4080
   caacactgtc ccccacgcta tcccccctgt ccccgacaat gtccccactg tttcctcctg 4140
   ttccctccta tccctgacac tgtccgccat gctgtcccca cctgtccctg acactgtctc 4200
   ccactctgtc ccctataatc cctgacactg tcccccacgc cgtcccctcc cgtatgcacc 4260
   actgtccccc aagctgtccc cacctgtcct caacacagtc ccccatgctg tccccacctg 4320
   tccccaacac tctcctccat gtccccacct gtccctgata ttgtccccca tgcagtcccc 4380
   acctgtcccc gatgctgtcc cccgggctgt acctaccagt ccaacactgt cccccacact 4440
   ctccccacct gtccctgata ctgtccccca tgctgtcccc acctgtcccg gacactgttc 4500
   tccacgctct cccctcctgt ccctgacact gtcccccaca ctgtccccac ctgtccccaa    4560
   cactatcctc catcctgtcc caacctgtct cctacactgt cccccatgct gtccccacca    4620
   gtccccaaca ctgtcctcca tgctgtcccc catgtcccca acactgtccc ccatgctatc    4680
   tcccctgtcc ctgacaatgt ccccactgtt tcctgtcccc tcctatccct gacactgtcc    4740
   cccatgctgt ccccacctgt cccccacatg gtctccaccg gtccctgaca ctgtctccca    4800
   ctctgtcccc tataatccct gacactgtcc cccacaccgt cccctcctgt atgcaccact    4860
   gtcccccatg ctgtccccac ctgtccctga tgctgtcctc cacacagtcc ccacctctcc    4920
   ctgacactgt ccccatctct ccccaacact ctcctccatg ctgtccttaa ctgtccccaa    4980
   cactcttcca cactctgtct ccacctgtcc ctgacactgt cccccacact gtcctcacct    5040
   gtgtctgaca ctgtccccca cgctgtcccc acctgtccct gacgctgtct tctgtgctgt    5100
   ccacatgctg ttggtgccct ggctctgctc tctatcacca agcctcagag caggcagtgg    5160
   tgaggccatg gcacctgggt ggcatgaggg gccggatggg cctcaggggc agggctgtgg    5220
   cctgcgtgga ctgacgggtg ggtgggcctt gggggcagag aggtggcctc agtgccctga    5280
   ggggtgggtg gggctcgggg gcagggctgt ggcctcgctc acccctgtgc tgtgccttgc    5340
   ctacaggtga agtggatctt ctcctcggtg gtggacctga agcagaccat catccccgac    5400
   tacaggaaca tgatcggaca gggggcctag ggccaccctc tgcggggtgt ccagggccgc    5460
   ccagacccca cacaccagcc atgggccatg ctcagccacc acccaggcca cacctgcccc    5520
   cgacctcacc gccctcaacc ccatgactct ctggcctcgc agttgccctc tgaccctgac    5580
   acacctgaca cgcccccctt ccagaccctg tgcatagcag gtctacccca gacctccgct    5640
   gcttggtgca tgcagggcac tgggggccag gtgtcccctc agcaggacgt ccttgccctc    5700
   cggaccacaa ggtgctcaca caaaaggagg cagtgaccgg tatcccaggc ccccacccag    5760
   gcaggacctc gccctggagc caaccccgtc cacgccagcc tcctgaacac aggcgtggtt    5820
   tccagatggt gagtgggagc gtcagccgcc aaggtaggga agccacagca ccatcaggcc    5880
   ctgttgggga ggcttccgag agctgcgaag gctcactcag acggccttcc tcccagcccg    5940
   cagccagcca gcctccattc cgggcactcc cgtgaactcc tgacatgagg aatgaggttg    6000
   ttctgatttc aagcaaagaa cgctgctctc tggctcctgg gaacagtctc agtgccagca    6060
   ccaccccttg gctgcctgcc cacactgctg gattctcggg tggaactgga cccgcaggga    6120
   cagccagccc cagagtccgc actggggaga gaaggggcca ggcccaggac actgccacct    6180
   cccacccact ccagtccacc gagatcactc agagaagagc ctgggccatg tggccgctgc    6240
   aggagcccca cagtgcaagg gtgaggatag cccaaggaag ggctgggcat ctgcccagac    6300
   aggcctccca gagaaggctg gtgaccaggt cccaggcggg caagactcag ccttggtggg    6360
   gcctgaggac agaggaggcc caggagcatc ggggagagag gtggagggac accgggagag    6420
   ccaggagcgt ggacacagcc agaactcatc acagaggctg gcgtccagcc ccgggtcacg    6480
   tgcagcagga acaagcagcc actctggggg caccaggtgg agaggcaaga cgacaaagag    6540
   ggtgcccgtg ttcttgcgaa agcagggctg ctggccacga gtgctggaca gaggccccca    6600
   cgctctgctg cccccatcac gccgttccgt gactgtcacg cagaatctgc agacaggaag   6660
   ggagactcga gcgggagtgc ggccagcgcc tgcctcggcc gtcagggagg actcctgggc   6720
   tcactcgaag gaggtgccac catttcagct ttggtagctt ttcttcttct tttaaatttt   6780
   ctaaagctca ttaattgtct ttgatgtttc ttttgtgatg acaataaaat atccttttta   6840
   agtcttgtac ttcgtgatgg gagc                                          6864
<210> 14
   <211> 21
   <212> DNA
   <213> Artificial
<220> primer
   <223> UpstreamSpe I Smu
<400> 14
   gagtaccgtt gtctgggtca c 21
<210> 15
   <211> 23
   <212> DNA
   <213> Artificial
<220> primer
   <223> SacI-3'Imu
<400> 15
   gagctctatg attattggtt aac 23
<210> 16
   <211> 22
   <212> DNA
   <213> Artificial
<220> primer
   <223> NeoI
<400> 16
   gcatgatctg gacgaagagc at 22
<210> 17
   <211> 22
   <212> DNA
   <213> Artificial
<220> primer
   <223> Neo2
<400> 17
   tcccctcaga agaactcgtc aa 22